# EUROPEAN PATENT APPLICATION

(11) **EP 1 516 903 A1**
(43) Date of publication of application: **23.03.2005**
(21) Application number: 04022456.0
(22) Date of filing: 21.09.2004
(51) Int. Cl.: C09K 11/06, H05B 33/14, H01L 51/20, H01L 51/30, C08F 26/12, C07D 413/14

(54) **Light-emitting compound and polymer and luminescent element**

(30) Priority: 22.09.2003 JP 2003330594; 28.06.2004 JP 2004190451
(71) Applicant: HIROSE ENGINEERING CO., LTD., Tokyo 146-0092 (JP)
(72) Inventor: Nakaya, Tadao, Ohta-ku Tokyo 146-0092 (JP); Matsumoto, Ryoji, Ohta-ku Tokyo 146-0092 (JP); Ishitobi, Tatsuro, Ohta-ku Tokyo 146-0092 (JP)
(74) Representative: Olgemöller, Luitgard, Dr.

(57) **Abstract**

The present invention provides a light-emitting compound and a light-emitting polymer capable of emitting white light themselves, and a luminescent element including them. The compound has the structure represented by general formula (1). 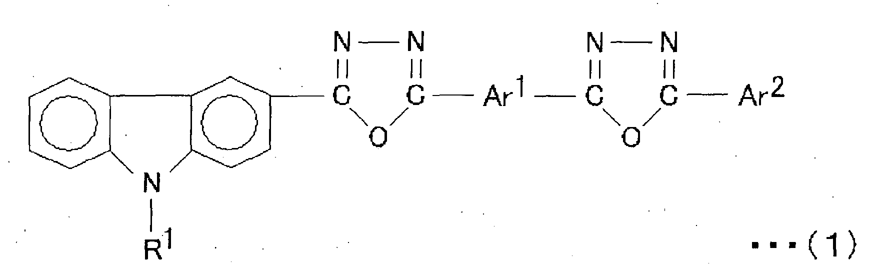 The polymer has the repeating unit represented by formula (3).

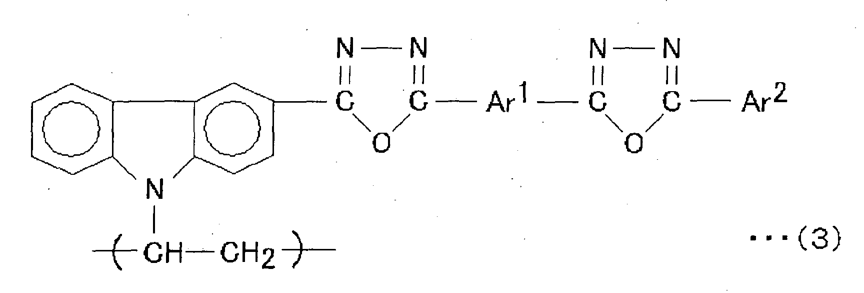

## Description

### Technical Field

The present invention relates to a light-emitting compound, a light-emitting polymer, and luminescent elements. More particularly, this invention relates to a light-emitting compound and polymer capable of emitting light at a high luminance upon the application of electric energy and being excellent in durability, and luminescent elements utilizing them.

### Background Art

Conventional technologies have not seen organic compounds that emit light at a high luminance. Needless to say, there were no organic compounds capable of emitting light of high purity at a high luminance upon the application of electric energy and being excellent in durability.

### Summary of the Invention

One objective of the present invention is to provide an organic compound and polymer which can emit a light of a color with a high purity at a high luminance and be excellent in durability. Another objective of the present invention is to provide luminescent elements which can emit a light of a color with a high purity at a high luminance and be excellent in durability.

In order to achieve the first objective, the present invention provides a light-emitting compound having the structure represented by formula (1). 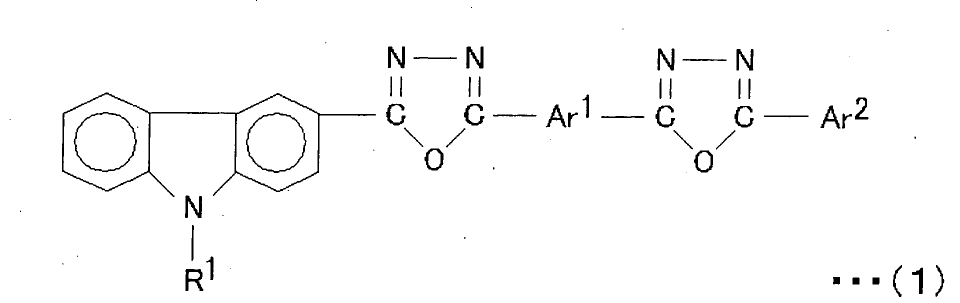 In formula (1), R¹ is hydrogen atom, a vinyl group, an aryl group, or an alkyl group having 1 to 10 carbon atoms, hydrogen atoms of which may be replaced with halogen atoms; Ar¹ denotes a group made by eliminating two hydrogen atoms from an aromatic ring to which the hydrogen atoms are bonded; and Ar² denotes an aryl group.

A preferable embodiment of the first aspect of the invention provided by the present invention to achieve the first objective is a light-emitting compound having the structure represented by formula (2). 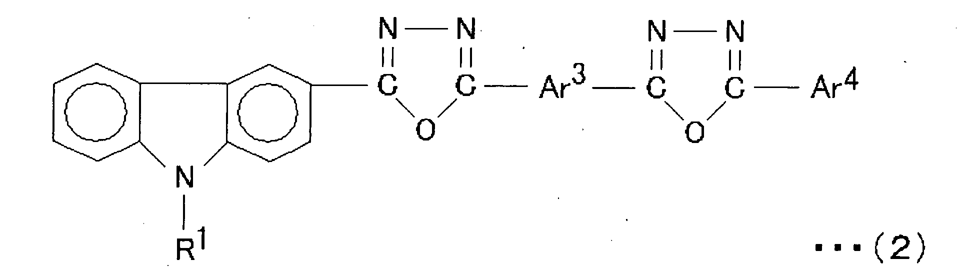 In formula (2), R¹ means the same group as that defined in the explanation of formula (1); Ar³ denotes the substituent shown by formula (2-1) or (2-2) below; and Ar⁴ is an aryl group represented by formula (2-3) or (2-4) below.

Formula (2-1) is: 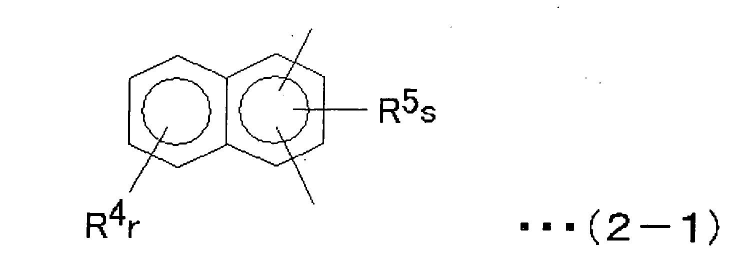 wherein each of R⁴ and R⁵ means hydrogen atom, or an alkyl group having 1 to 10 carbon atoms, hydrogen atoms of which may be replaced with halogen atoms; r denotes an integer of 1 to 4; and s denotes an integer of 1 or 2, wherein R⁴ and R⁵ may be the same or different from each other; R⁴s, the number of which number is r, may be the same or different from each other when r is not 1; and two R⁵s, when s is 2, may be the same or different from each other.

Formula (2-2) is: 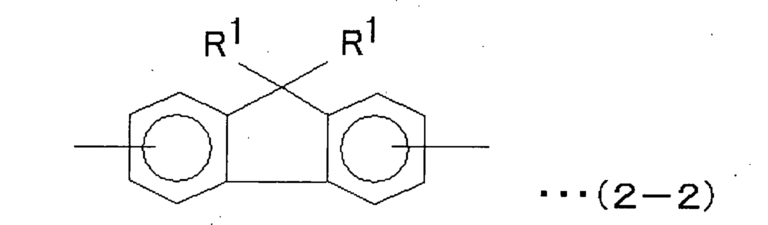 wherein R¹ means the same group as that defined in the explanation of formula (1) above; and two R¹s may be the same or different from each other.

Formula (2-3) is: 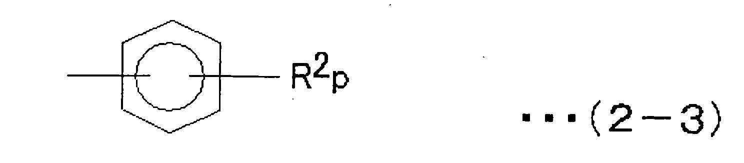 wherein R² means hydrogen atom, or an alkyl group having 1 to 10 carbon atoms, hydrogen atoms of which may be replaced with halogen atoms; p denotes an integer of 1 to 5; and R²s, the number of which is p, may be the same or different from each other when p is not 1.

Formula (2-4) is: 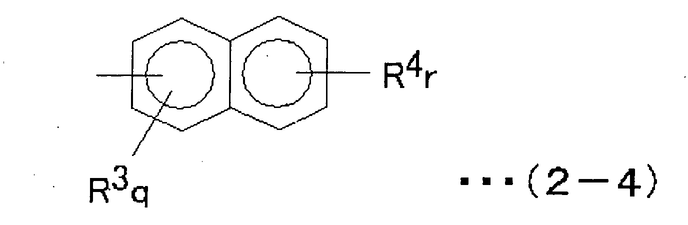 wherein R³ means hydrogen atom, or an alkyl group having 1 to 10 carbon atoms, hydrogen atoms of which may be replaced with halogen atoms; q denotes an integer of 1 to 3; R⁴ and r are the same as those defined in the explanation of formula (2-1) above; R³s, the number of which is q, may be the same or different from each other when q is not 1; and R⁴s, the number of which is r, may be the same or different from each other when r is not 1.

The second aspect of the invention provided by the present invention to achieve the first objective is a light-emitting polymer having the repeating unit represented by formula (3). 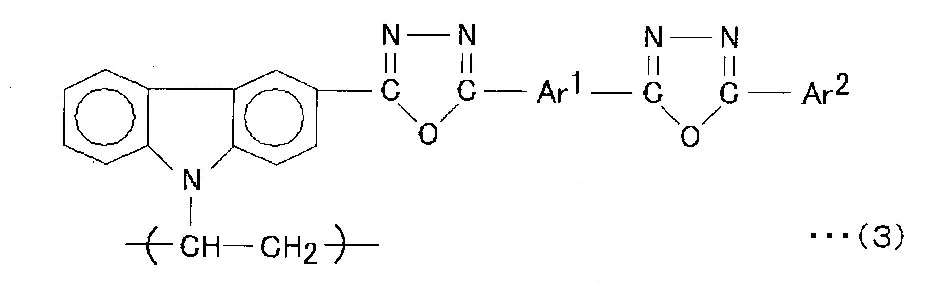 In formula (3), Ar¹ and Ar² mean the same as those defined in the explanation of formula (1) above.

The third aspect of the invention provided by the present invention to achieve the second objective is a luminescent element having a pair of electrodes and a light-emitting layer sandwiched between the electrodes, the light-emitting layer including the light-emitting compound represented by formula (1) or (2), or the light-emitting polymer having the repeating unit represented by formula (3).

This invention can provide a light-emitting compound and a light-emitting polymer having excellent properties such as high luminance and enhanced durability, an easy method for producing them, and luminescent elements having excellent properties such as high luminance and enhanced durability.

### Brief Description of the Drawings

Figure 1 is an illustration showing an example of the luminescent element in accordance with the present invention.
Figure 2 is an illustration showing another example of the luminescent element in accordance with the present invention.
Figure 3 is an illustration showing a still another example of the luminescent element in accordance with the present invention.
Figure 4 is an illustration showing a further example of the luminescent element in accordance with the present invention.
Figure 5 is an NMR spectrum chart of the compound represented by formula (6) in Example 1.
Figure 6 is an IR spectrum chart of the compound represented by formula (6) in Example 1.
Figure 7 is an NMR spectrum chart of the compound represented by formula (7) in Example 1.
Figure 8 is an IR spectrum chart of the compound represented by formula (7) in Example 1.
Figure 9 is an NMR spectrum chart of the compound represented by formula (9) in Example 1.
Figure 10 is an IR spectrum chart of the compound represented by formula (9) in Example 1.
Figure 11 is an NMR spectrum chart of the compound represented by formula (10) in Example 1.
Figure 12 is an IR spectrum chart of the compound represented by formula (10) in Example 1.
Figure 13 is an NMR spectrum chart of the compound represented by formula (11) in Example 1.
Figure 14 is an IR spectrum chart of the compound represented by formula (11) in Example 1.
Figure 15 is a fluorescence spectrum chart of the compound represented by formula (11) in Example 1.
Figure 16 is an NMR spectrum chart of the compound represented by formula (12) in Example 2.
Figure 17 is an IR spectrum chart of the compound represented by formula (12) in Example 2.
Figure 18 is an NMR spectrum chart of the compound represented by formula (14) in Example 2.
Figure 19 is an IR spectrum chart of the compound represented by formula (14) in Example 2.
Figure 20 is an NMR spectrum chart of the compound represented by formula (15) in Example 2.
Figure 21 is an IR spectrum chart of the compound represented by formula (15) in Example 2.
Figure 22 is an NMR spectrum chart of the compound represented by formula (16) in Example 2.
Figure 23 is an IR spectrum chart of the compound represented by formula (16) in Example 2.
Figure 24 is an NMR spectrum chart of the compound represented by formula (17) in Example 2.
Figure 25 is an IR spectrum chart of the compound represented by formula (17) in Example 2.
Figure 26 is an NMR spectrum chart of the compound represented by formula (18) in Example 2.
Figure 27 is an IR spectrum chart of the compound represented by formula (18) in Example 2.
Figure 28 is an NMR spectrum chart of the compound having the repeating unit represented by formula (19) in Example 2.
Figure 29 is an IR spectrum chart of the compound having the repeating unit represented by formula (19) in Example 2.
Figure 30 is a fluorescence spectrum chart of the compound having the repeating unit represented by formula (19) in Example 2.
Figure 31 is an NMR spectrum chart of the compound represented by formula (20) in Example 3.
Figure 32 is an IR spectrum chart of the compound represented by formula (20) in Example 3.
Figure 33 is an NMR spectrum chart of the compound represented by formula (21) in Example 4.
Figure 34 is an IR spectrum chart of the compound represented by formula (21) in Example 4.
Figure 35 is an NMR spectrum chart of the compound represented by formula (22) in Example 4.
Figure 36 is an IR spectrum chart of the compound represented by formula (22) in Example 4.
Figure 37 is an NMR spectrum chart of the compound represented by formula (23) in Example 4.
Figure 38 is an IR spectrum chart of the compound represented by formula (23) in Example 4.
Figure 39 is an NMR spectrum chart of the compound represented by formula (24) in Example 4.
Figure 40 is an IR spectrum chart of the compound represented by formula (24) in Example 4.
Figure 41 is an NMR spectrum chart of the compound represented by formula (25) in Example 4.
Figure 42 is an IR spectrum chart of the compound represented by formula (25) in Example 4.
Figure 43 is an NMR spectrum chart of the compound represented by formula (26) in Example 4.
Figure 44 is an IR spectrum chart of the compound represented by formula (26) in Example 4.
Figure 45 is a fluorescence spectrum chart of the compound having the repeating unit represented by formula (26) in Example 4.

### Detailed Description of the Preferred Embodiments

The light-emitting compound according to the present invention is characterized by having the structure represented by formula (1).

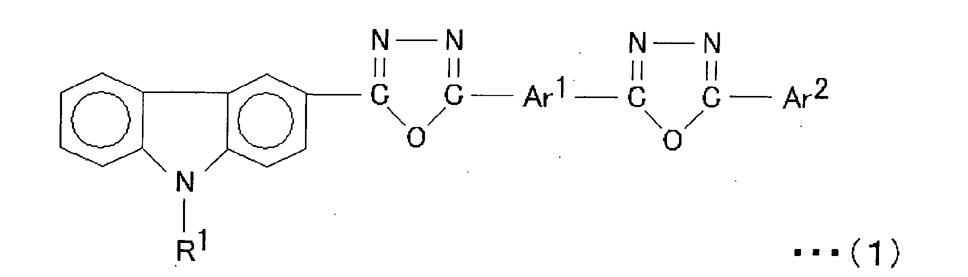

The compound represented by formula (1) has a carbazole skeleton, the nitrogen atom of which is bonded with R¹. Also, the carbon atom at the 3-position or the 6-position is bonded with one of the carbon atoms belonging to the first 1,3,4-oxadiazole. The other carbon atom of the first 1,3,4-oxadiazole is bonded with the group represented by Ar¹ in formula (1), which is made by eliminating two hydrogen atoms from the aromatic ring to which the hydrogen atoms are bonded.

The group Ar¹ is also bonded with another 1, 3, 4-oxadiazole. The carbon atom of this second 1,3,4-oxadiazole that is not bonded with Ar¹ is bonded with Ar².

R¹ is hydrogen atom, a vinyl group, an alkyl group having 1 to 10 carbon atoms, hydrogen atoms of which may be replaced with halogen atoms, or an aryl group represented by formula (1-1), (1-2), (1-3), (1-4), (1-5), or (1-6).

Examples of the alkyl group having 1 to 10 carbon atoms, hydrogen atoms of which are not replaced with halogen atoms, are methyl group, ethyl group, propyl group, isopropyl group, n-butyl group, isobutyl group, sec-butyl group, tert-butyl group, n-pentyl group, sec-pentyl group, tert-pentyl group, n-hexyl group, n-heptyl group, an octyl group, a nonyl group, a decyl group, etc. Among those are preferred an alkyl group having 1 to 5 carbon atoms, such as methyl group, ethyl group, propyl group, isopropyl group, n-butyl group, isobutyl group, sec-butyl group, tert-butyl group, n-pentyl group, sec-pentyl group, or tert-pentyl group. Methyl group, ethyl group, or propyl group is especially preferable.

The halogen atom includes fluorine atom, chlorine atom, or bromine atom.

Examples of the alkyl group having 1 to 10 carbon atoms, hydrogen atoms of which are replaced with halogen atoms, are fluoromethyl group, difluoromethyl group, trifluoromethyl group, fluoroethyl group, 1,1-difluoroethyl group, 1,2-difluoroethyl group, 1,1,1-trifluoroethyl group, 1,1,2-. trifluoroethyl group, 1,2,2-trifluoroethyl group, 1,1,2,2-tetrafluoroethyl group, 1,1,2,2,2-pentafluoroethyl group, 1-fluoropropyl group, 2-fluoropropyl group, 1,1-difluoropropyl group, 1,2-difluoropropyl group, 1,3-difluoropropyl group, 2,2-difluoropropyl group, 1,1,1-trifluoropropyl group, 1,1,2-trifluoropropyl group, 1,2,3-trifluoropropyl group, 1,2,2-trifluoropropyl group, 1,3,3-trifluoropropyl group, chloromethyl group, dichloromethyl group, trichloromethyl group, chloroethyl group, 1,1-dichloroethyl group, 1,2-dichloroethyl group, 1,1,1-trichloroethyl group, 1,1,2-trichloroethyl group, 1,2,2-trichloroethyl group, 1,1,2,2-tetrachloroethyl group, 1,1,2,2,2-pentachloroethyl group, 1-chloropropyl group, 2-chloropropyl group, 1,1-dichloropropyl group, 1,2-dichloropropyl group, 1,3-dichloropropyl group, 2,2-dichloropropyl group, 1,1,1-trichloropropyl group, 1,1,2-trichloropropyl group, 1,2,3-trichloropropyl group, 1,2,2-trichloropropyl group, 1,3,3-trichloropropyl group, bromomethyl group, dibromomethyl group, tribromomethyl group, bromoethyl group, 1,1-dibromoethyl group, 1,2-dibromoethyl group, 1,1,1-tribromoethyl group, 1,1,2-tribromoethyl group, 1,2,2-tribromoethyl group, 1,1,2,2-tetrabromoethyl group, 1,1,2,2,2-pentabromoethyl group, 1-bromopropyl group, 2-bromopropyl group, 1,1-dibromopropyl group, 1,2-dibromopropyl group, 1,3-dibromopropyl group, 2, 2-dibromopropyl group, 1,1,1-tribromopropyl group, 1,1,2-tribromopropyl group, 1,2,3-tribromopropyl group, 1,2,2-tribromopropyl group, and 1,3,3-tribromopropyl group.

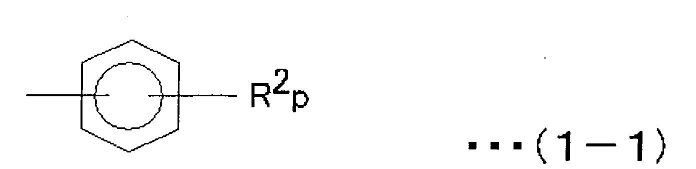

The aryl group shown by formula (1-1) has a phenyl skeleton, which has at least one R².

R² is hydrogen atom, or an alkyl group having 1 to 10 carbon atoms, hydrogen atoms of which may be replaced with halogen atoms. Examples of the alkyl group are the same as those of the alkyl group included in R¹ above.

In the formula, p means an integer of 1 to 5. When p is 2 to 5, R²s may be the same or different from each other.

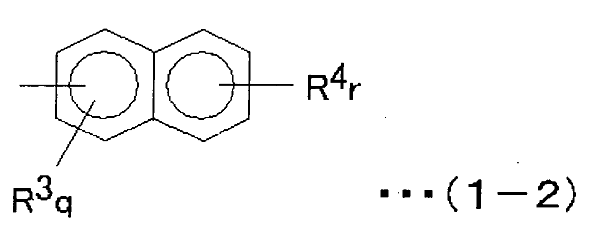

The aryl group represented by formula (1-2) has a naphthyl skeleton, and has R³ and R⁴.

R³ and R⁴ each means hydrogen atom, or an alkyl group having 1 to 10 carbon atoms, hydrogen atoms of which may be replaced with halogen atoms. Examples of the alkyl group are the same as those of the alkyl group included in R¹ above.

In formula (1-2), q denotes an integer of 1 to 3, and r an integer of 1 to 4. R³ and R⁴ may be the same or different from each other. Also, when q is not 1, R³s may be the same or different. The same can be applied to R⁴.

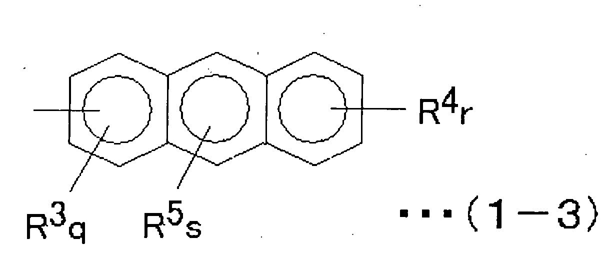

The aryl group represented by formula (1-3) has an anthryl skeleton made by eliminating one of the hydrogen atoms respectively at the 1-, 2-, 3-, 4-, 5-, 6-, 7-, and 8-positions, and has R³, R⁴ and R⁵.

Concerning R³ and R⁴, the respective definitions are given above. R⁵ is hydrogen atom, or an alkyl group having 1 to 10 carbon atoms, hydrogen atoms of which may be replaced with halogen atoms. Examples of the alkyl group are the same as those of the alkyl group included in R¹ above.

In the formula, q and r are the same as those above. s denotes an integer of 1 or 2. R³, R⁴ and R⁵ may be the same or different from each other. Also, when q is not 1, R³s may be the same or different. The same can be applied to R⁴ and R⁵.

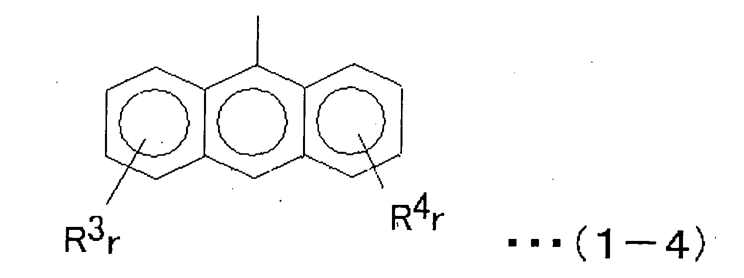

The aryl group represented by formula (1-4) has an anthryl skeleton made by eliminating one of the hydrogen atoms respectively at the 9- and 10-positions, and has R³ and R⁴.

With respect to R³, R⁴ and r, the respective definitions are given above. R³ and R⁴ may be the same or different from each other. Also, when r is not 1, R³s may be the same or different. The same can be applied to R⁴.

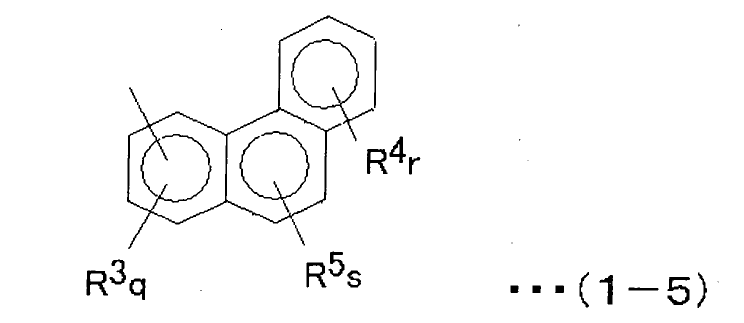

The aryl group represented by formula (1-5) has a phenanthryl skeleton made by eliminating one of the hydrogen atoms respectively at the 1-, 2-, 3-, 4-, 5-, 6-, 7-, and 8-positions, and has R³, R⁴ and R⁵.

With respect to R³, R⁴, R⁵, q, r, and s in formula (1-5), their definitions are given above. R³, R⁴ and R⁵ may be the same or different from each other. Also, when q is not 1, R³s may be the same or different. The same can be applied to R⁴ and R⁵.

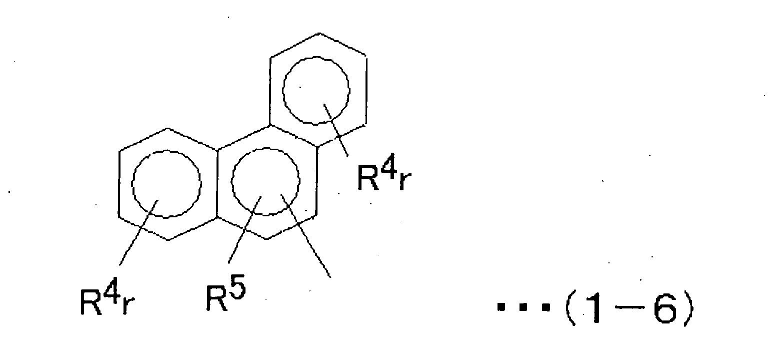

The aryl group represented by formula (1-6) has a phenanthryl skeleton made by eliminating one of the hydrogen atoms at the 9- and 10-positions, and has R⁴ and R⁵.

With respect to R⁴, R⁵, and r in formula (1-6), their definitions are given above. R⁴ and R⁵ may be the same or different from each other. Also, when r is not 1, R⁴s may be the same or different.

Ar¹ in formula (1) means one of the groups represented by formulas (1-1') to (1-10').

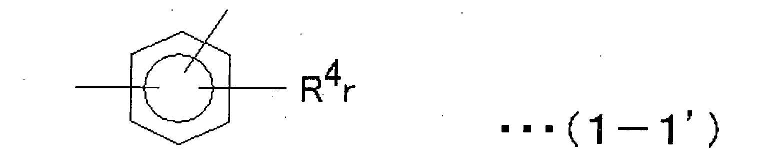

The group shown by formula (1-1') has a phenylene skeleton made by eliminating two hydrogen atoms respectively bonded to the benzene ring, and at least one R⁴.

R⁴ and r in formula (1-1') have been defined above. When r is not 1, R⁴s may be the same or different from each other.

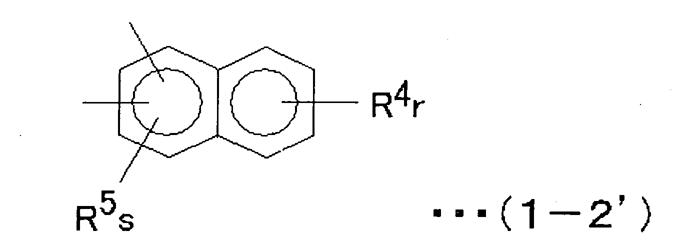

The group shown by formula (1-2') has a naphthylene skeleton made by eliminating two hydrogen atoms, each of which is bonded to the naphthalene ring at one of the 1-, 2-, 3-, and 4-positions, and R⁴ and R⁵. R⁴, R⁵, r, and s in formula (1-2') have been defined above. R⁴ and R⁵ may be the same of different from each other. When r is not 1, R⁴s may be the same or different from each other. Also, when s is not 1, R⁵s may be the same or different from each other.

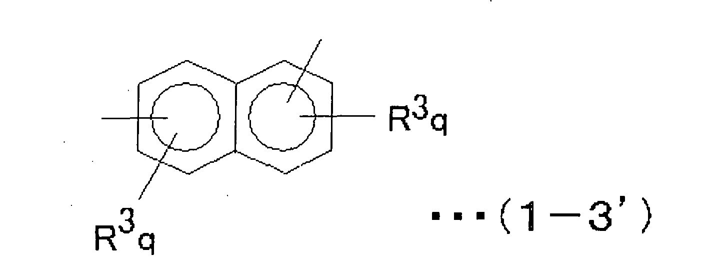

The group shown by formula (1-3') has a naphthylene skeleton made by eliminating a hydrogen atom bonded to the naphthalene ring at the 1-, 2-, 3-, or 4-position and another hydrogen atom at the 5-, 6-, 7-, or 8-position, and R³. R³ and q in formula (1-3') have been defined above. R³ at one benzene moiety of the skeleton and that at the other may be the same of different from each other. When q is not 1, R³s bonded to the same benzene moiety may be the same or different from each other.

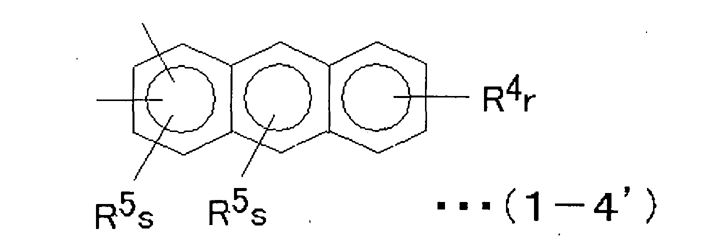

The group shown by formula (1-4') has an anthrylene skeleton made by eliminating two hydrogen atoms, each of which is bonded to the anthracene ring at one of the 1-, 2-, 3-, or 4-position, and R⁴ and R⁵. R⁴, R⁵, r, and s in formula (1-4') have been defined above. R⁴, one R⁵ at one benzene moiety, and the other R⁵ at the other benzene moiety may be the same or different from each other. When r is not 1, R⁴s may be the same or different from each other. Also, when s is not 1, R⁵s bonded to the same benzene moiety may be the same or different from each other.

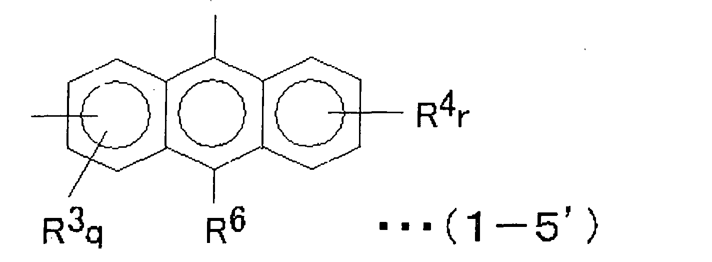

The group shown by formula (1-5') has an anthrylene skeleton made by eliminating a hydrogen atom bonded to the anthracene ring at one of the 1-, 2-, 3-, and 4-positions and another hydrogen atom at the 9-position, and R³, R⁴ and R⁶. R³, R⁴, q, and r in formula (1-5') have been defined above. R³, R⁴ and R⁶ may be the same of different from each other. When q is not 1, R³s may be the same or different from each other. Also, when r is not 1, R⁴s may be the same or different from each other.

R⁶ is hydrogen atom or an alkyl group having 1 to 10 carbon atoms. The alkyl group is the same as that explained above.

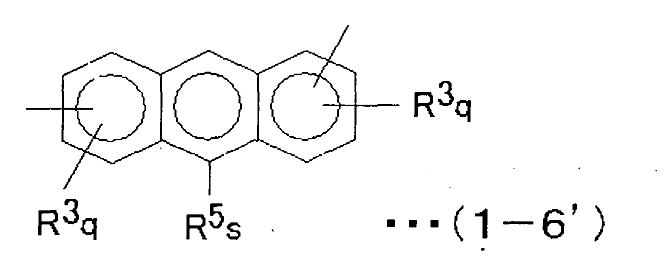

The group shown by formula (1-6') has an anthrylene skeleton made by eliminating a hydrogen atom bonded to the anthracene ring at one of the 1-, 2-, 3-, and 4-positions and another hydrogen atom at one of the 5-, 6-, 7-, and 8-positions, and R³ and R⁵. R³, R⁵, q, and s in formula (1-6') have been defined above. One R³ at one benzene moiety, the other R³ at the other benzene moiety, and R⁵ may be the same of different from each other. When q is not 1, R³s bonded to the same benzene moiety may be the same or different from each other. Also, when s is not 1, R⁵s may be the same or different from each other.

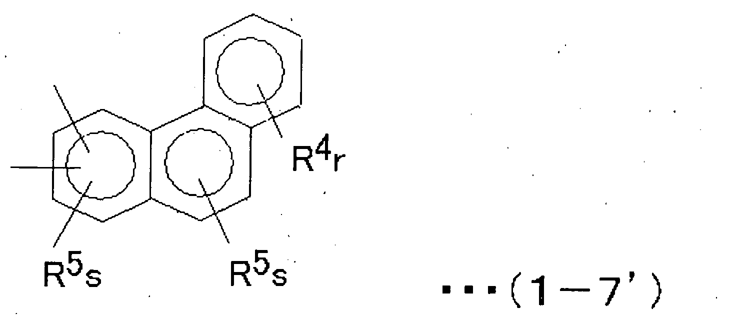

The group shown by formula (1-7') has a phenanthylene skeleton made by eliminating two hydrogen atoms, each of which is bonded to the phenanthrene ring at one of the 1-, 2-, 3-, or 4-position, and R⁴ and R⁵. R⁴, R⁵, r, and s in formula (1-7') have been defined above. R⁴, one R⁵ at one benzene moiety, and the other R⁵ at the other benzene moiety may be the same of different from each other. When r is not 1, R⁴s may be the same or different from each other. Also, when s is not 1, R⁵s bonded to the same benzene moiety may be the same or different from each other.

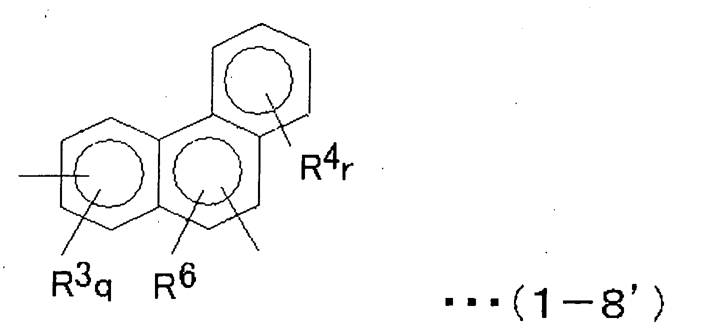

The group shown by formula (1-8') has a phenanthylene skeleton made by eliminating a hydrogen atom bonded to the anthracene ring at one of the 1-, 2-, 3-, and 4-positions and another hydrogen atom at one of the 9- and 10-positions, and R³, R⁴ and R⁶. R³, R⁴, R⁶, q, and r in formula (1-8') have been defined above. R³, R⁴ and R⁶ may be the same or different from each other. When q is not 1, R³s may be the same or different from each other. Also, when r is not 1, R⁴s may be the same or different from each other.

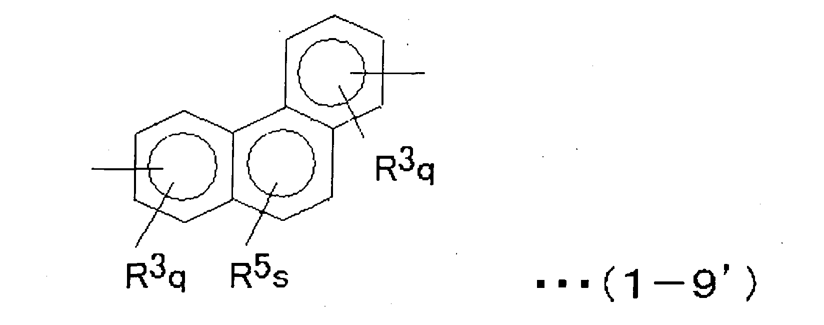

The group shown by formula (1-9') has a phenanthylene skeleton made by eliminating a hydrogen atom bonded to the phenanthrene ring at one of the 1-, 2-, 3-, and 4-positions and another hydrogen atom at one of the 5-, 6-, 7-, and 8-positions, and R³ and R⁵. R³, R⁵, q, and s in formula (1-6') have been defined above. One R³ at one benzene moiety, the other R³ at the other benzene moiety, and R⁵ may be the same of different from each other. When q is not 1, R³s bonded to the same benzene moiety may be the same or different from each other. Also, when s is not 1, R⁵s may be the same or different from each other.

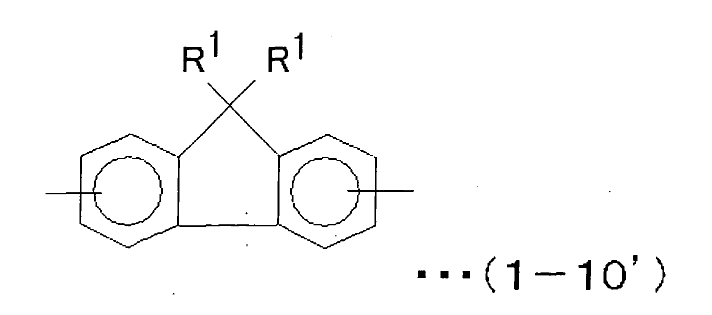

The group shown by formula (1-10') has a fluorylene skeleton made by eliminating a hydrogen atom bonded to the fluorene ring at one of the 1-, 2-, 3-, and 4-positions and another hydrogen atom at one of the 5-, 6-, 7-, and 8-positions, and the carbon atom at the 9-position has two R¹s. R¹ has been defined above. Two R¹s may be the same of different from each other.

Ar² in formula (1) is an aryl group represented by one of formulas (1-1) to (1-6).

The first light-emitting compound represented by formula (1) may be prepared by Reaction Process 1 below. In this process, R¹, Ar¹ and Ar² mean the same as those defined above.

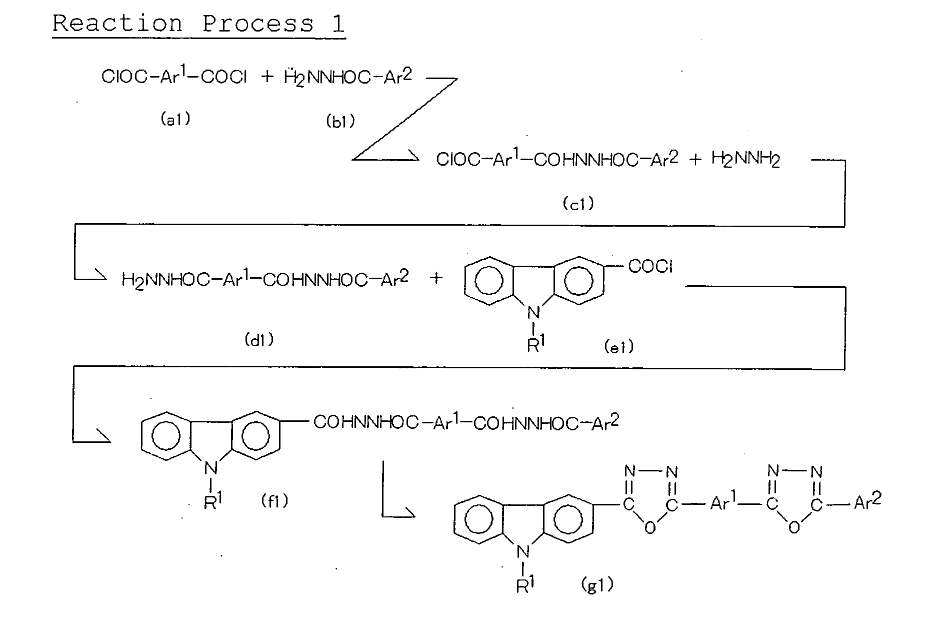

A mixture of an acid chloride (a1) and a hydrazide compound (b1), in the amount ratio of the former to the latter being 1:1, is heated in a solvent, and a compound (c1) is obtained.

The solvent may be a non-polar solvent, or a polar solvent suchasortho-dichlorobenzene,meta-dichlorobenzene, pyridine, dioxane, N,N-dimethylformamide, or tetrahydrofuran (THF).

The reaction temperature should be 50 to 80°C, particularly 60 to 70°C.

The heating time should be 2 to 6 hours, particularly 3 to 4 hours.

Then, a carbohydrazide (d1) may be prepared by heating the compound (c1) and hydrazine in a solvent.

The amount of hydrazine to that of the compound (c1) should be excessive, but the former should be up to 10 times as much as the latter.

The solvent may be a non-polar solvent, or a polar solvent such asortho-dichlorobenzene,meta-dichlorobenzene, pyridine, dioxane, N,N-dimethylformamide, or tetrahydrofuran (THF).

The reaction temperature should be 40 to 70°C, particularly 50 to 60°C.

The heating time should be at least 2 hours, particularly at least 4 hours.

Next, a compound (f1) may be prepared by heating the obtained carbohydrazide (d1) and a carbazole derivative (e1) in a solvent.

The solvent may be a non-polar solvent, or a polar solvent such as ortho-dichlorobenzene, meta-dichlorobenzene, pyridine, dioxane, N,N-dimethylformamide, or tetrahydrofuran (THF).

The reaction temperature should be 40 to 80°C, particularly 60 to 70°C.

The heating time should be 2 to 8 hours, particularly 4 to 6 hours.

Furthermore, the obtained compound (f1) is subjected to a ring-closing reaction in a heated solvent, which produces the first light-emitting compound represented by formula (1), the compound shown by (g1) in reaction process 1.

The solvent may be a non-polar solvent, or a polar solvent such as ortho-dichlorobenzene, meta-dichlorobenzene, pyridine, dioxane, N,N-dimethylformamide, or tetrahydrofuran (THF).

The reaction temperature should be 100 to 130°C, particularly 110 to 120°C.

The heating time should be 15 to 20 hours, particularly 17 to 19 hours.

The first light-emitting compound represented by formula (1) can finally be obtained through purification and separation by known methods after the completion of the series of reactions. The obtained compound can easily be identified by IR spectrum analysis, NMR spectrum analysis and fluorescence spectrum analysis.

Of the specific light-emitting compounds having the general structure represented by formula (1) are preferred those having the structure represented by formula (2).

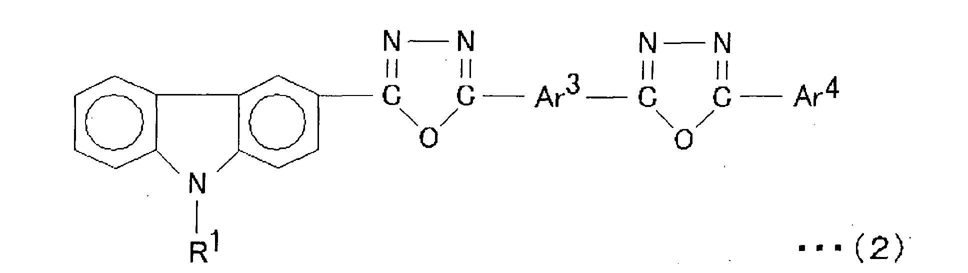

The compound represented by formula (2) has a carbazole skeleton, the nitrogen atom of which is bonded with R¹. Also, the carbon atom at the 3-position or the 6-position is bonded with one of the carbon atoms belonging to the first 1,3,4-oxadiazole. The other carbon atom of the first 1,3,4-oxadiazole is bonded with the group represented by Ar³ in formula (2), which is made by eliminating two hydrogen atoms from the aromatic ring to which the hydrogen atoms are bonded.

The group Ar³ is also bonded with another 1,3,4-oxadiazole. The carbon atom of this second 1,3,4-oxadiazole that is not bonded with Ar³ is bonded with Ar⁴.

R¹ is the same as R¹ in formula (1).

Ar³ is a group represented by formula (1-2') or (1-10') as defined above. Also, Ar⁴ is a group represented by formula (1-1) or (1-2) as defined above.

The preferable light-emitting compound represented by formula (2) may be prepared by Reaction Process 2 below. In this process, R¹, Ar³ and Ar⁴ mean the same as those defined above.

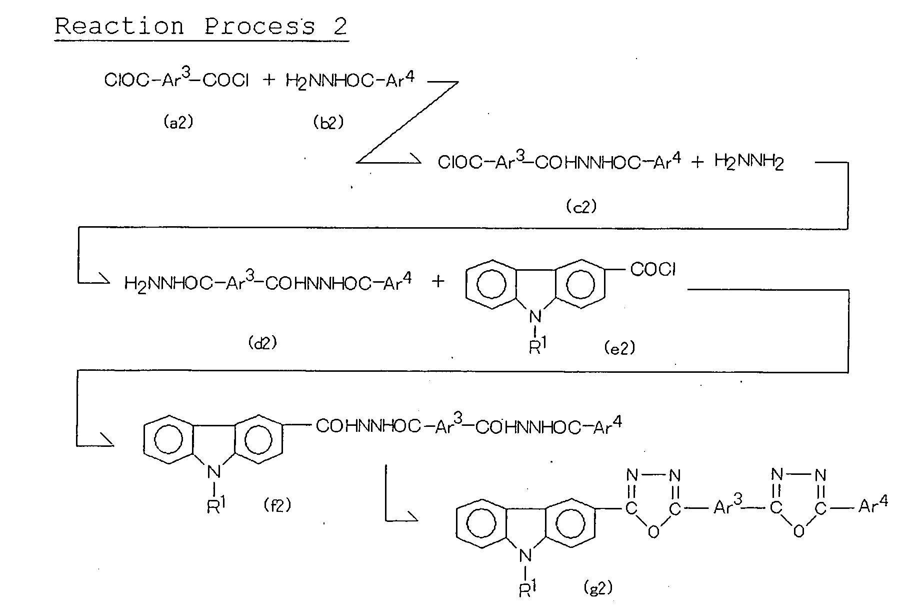

A mixture of an acid chloride (a2) and a hydrazide compound (b2), in the amount ratio of the former to the latter being 1:1, is heated in a solvent, and a compound (c2) is obtained. The reaction conditions, such as the reaction temperature and the heating time, are the same those of the corresponding step in Reaction Process 1 for preparing the light-emitting compound having the structure represented by formula (1).

Also, the reaction conditions for preparing a compound (g2) from the compound (c2) are same as those of the steps of preparing the compound (g1) from the compound (c1).

The light-emitting polymer in accordance with the present invention is characterized by the repeating unit represented by formula (3).

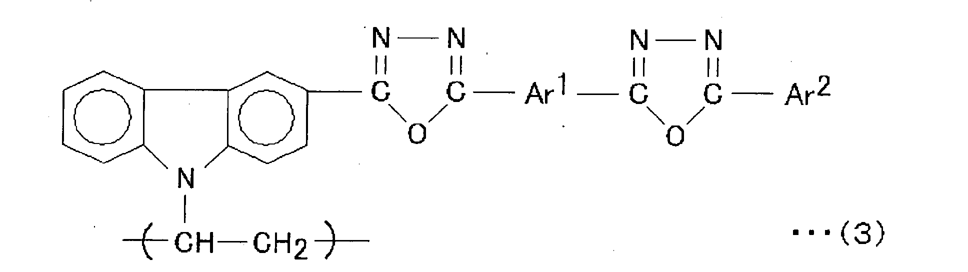

Although the light-emitting polymer having the repeating unit of formula (3) has a structure similar to that of the light-emitting compound of formula (1), the difference between them is that the latter has a carbazole skeleton while the former has a N-polyvinylcarbazole skeleton.

Ar¹ and Ar² in formula (3) are the same as those in formula (1).

The light-emitting polymer having the repeating unit represented by formula (3) may be produced by Reaction Process 3 below. In the process, Ar¹ and Ar² are the same as those in formula (1).

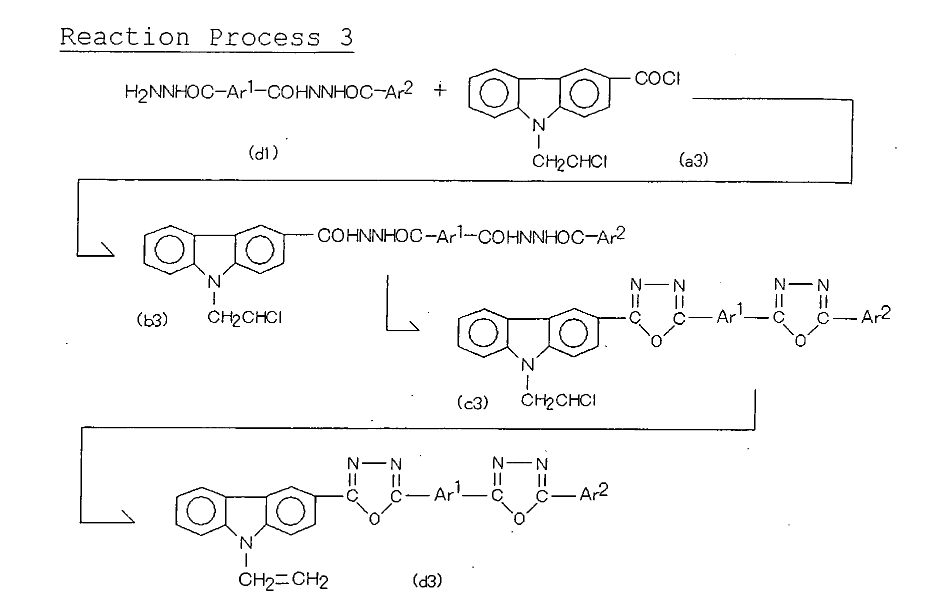

The reaction conditions for the steps of preparing a compound (c3) from the carbohydrazide (d1) and the carbazole derivative (a3) are the same as those for the corresponding steps in Reaction Process 1 or 2 where the light-emitting compounds (g1) or (g2) is produced.

Heating the obtained compound (c3) in a solvent makes a dehydrochloride reaction take place, which results in a compound (d3).

The solvent may be a non-polar solvent, or a polar solvent such as methanol, ethanol, ortho-dichlorobenzene, meta-dichlorobenzene, pyridine, dioxane,N,N-dimethylformamide, or tetrahydrofuran (THF).

The reaction temperature should be 80 to 110°C, particularly 90 to 100°C.

The heating time should be 2 to 6 hours, particularly 3 to 4 hours.

After the addition of a polymerization initiator, the compound (d3) is heated and made to polymerize to the light-emitting polymer of the present invention, which is a polymer having the repeating unit (e3) shown in Reaction Process 4 below.

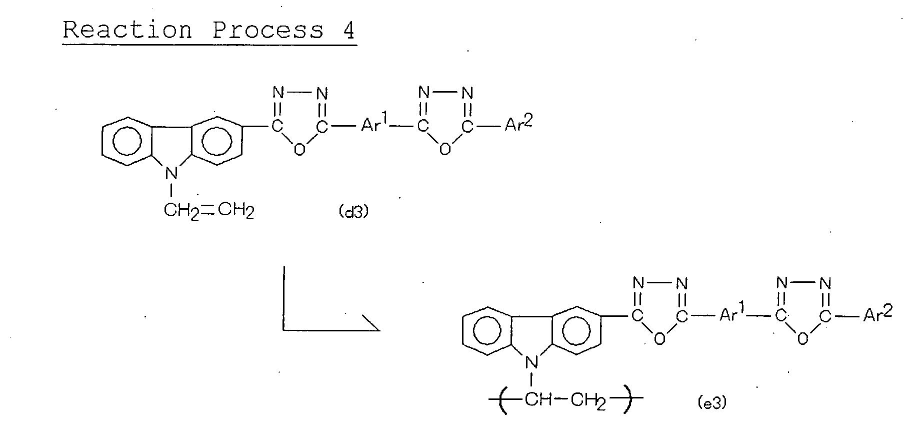

The solvent may be a non-polar solvent, or a polar solvent suchasortho-dichlorobenzene,meta-dichlorobenzene, pyridine, dioxane, N,N-dimethylformamide, or tetrahydrofuran (THF).

Knownpolymerizationinitiatorssuchasazobisisobutyronitrile or benzoyl peroxide may be used for the initiator in this process.

The reaction temperature should be at least 120°C, particularly 130 to 150°C.

The heating time should be 40 to 55 hours.

The light-emitting polymer having the repeating unit represented by formula (3) can finally be obtained through purification and separation by known methods after the completion of the series of reactions. The obtained polymer can easily be identified by IR spectrum analysis, NMR spectrum analysis and fluorescence spectrum analysis.

The light-emitting compounds and polymer according to the present invention can easily be produced simply by heating. Therefore it can be said that these simple processes of producing the light-emitting compounds and polymer are industrial methods.

The luminescent element utilizing the light-emitting compound or polymer of the present invention will be explained in the followings.

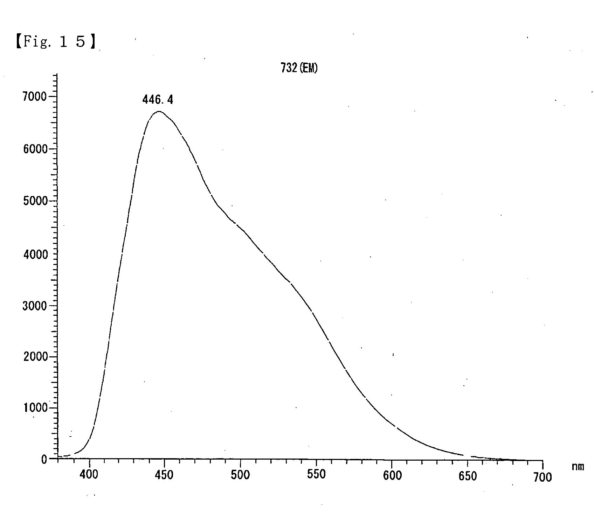

Upon the application of electromagnetic wave energy, it is observed that the compounds and polymer of the present invention emit a visible light of which wavelength ranges 420 nm and 600 nm. They have a fluorescence spectrum, for example as shown in Figure 15, and may be utilized for an organic electroluminescent element.

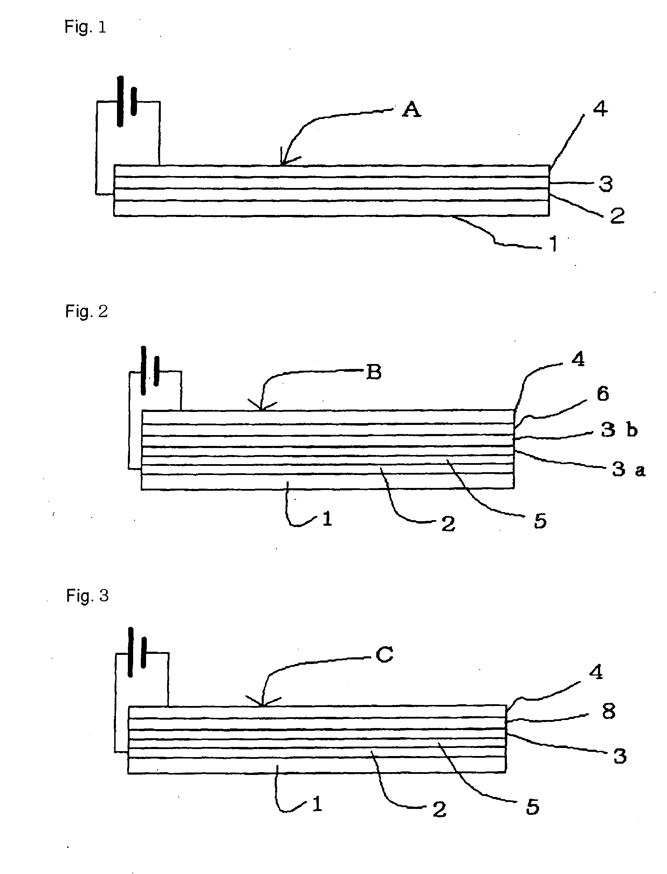

Figure 1 is a schematic illustration that shows the sectional structure of a luminescent element according to the present invention, which is a one-layer type organic EL element. As shown in this figure, a luminescent element A is prepared by layering a light-emitting layer 3 and an electrode layer 4 in this order on a substrate 1 with which a transparent electrode 2 has been provided. The light-emitting layer 3 includes the light-emitting compound or polymer of the present invention.

When electric current is applied to the luminescent element shown in Figure 1, the light-emitting layer 3 of which includes the light-emitting compound or polymer of the present invention, at the transparent electrode 2 and the electrode layer 4, the element emits light in accordance with the chemical structure of the light-emitting compound or polymer included. Upon the application of an electric field between the transparent electrode 2 and the electrode layer 4, electrons are injected from the electrode layer 4 and positive holes from the transparent electrode 2. In the light-emitting layer 3, the electrons are recombined with positive holes, which causes the energy level to return to the valence band from the conduction band. This transition of the energy level is accompanied by emission of the energy differential as light.

The luminescent element A shown in Figure 1, when it is shaped to a planar one with a large area, may be used as a planar illuminator, for example a large-area wall illuminator when fixed on a wall, or a large-area ceiling illuminator when fixed on a ceiling. This luminescent element may be utilized for a planar light source in place of a point light source, such as a conventional bulb, and a line light source, such as a conventional fluorescent lamp. In particular, this illuminator can suitably be used to light up walls, ceilings and floors in dwelling rooms, offices and passenger trains, or to make them emit light. Moreover, this luminescent element Amaybe suitable for the backlight used in displays of computers, cellular phones and ATMs. Furthermore, this illuminator may be used for various light sources, such as the light source of direct illumination and that of indirect illumination. Also, it may be used for the light sources of advertisement apparatuses, road traffic sign apparatuses and light-emitting billboards, which have to emit light at night and provide good visibility. In addition, because the luminescent element A includes a light-emitting compound or polymer having the special structure in the light-emitting layer, it has a long life. Therefore, light sources employing the luminescent element A will naturally have a long life.

The luminescent element Amay also be shaped into a tubular light emitter comprising a tubularly shaped substrate 1, a transparent electrode 2 placed on the inside surface of the substrate 1, a light emitting layer 3 and an electrode layer 4 placed on the transparent electrode 2 in this order. Because this luminescent element A does not include mercury, it is an ecological light source andmaybe a substitute for conventional fluorescent lamps.

For the substrate 1 may be used any known substrate, as long as the transparent electrode 2 can be formed on the surface of the substrate. Examples of the substrate 1 are a glass substrate, a plastic sheet, a ceramic substrate, and a metal substrate of which surface is insulated, for example, through the formation of an insulating layer thereon.

For the transparent electrode 2, various materials may be employed, as long as their work functions are large, they are transparent, and they can function as a cathode and inject holes to the light-emitting layer 3 when voltage is applied thereto. Specifically, the transparent electrode 2 may be made of a transparent inorganic conductive material of ITO, In₂O₃, SnO₂, ZnO, CdO, etc. and derivatives thereof, or an electrically conductive high polymer such as polyaniline.

The transparent electrode 2 may be formed on the substrate 1 by chemical vapor phase deposition, spray pyrolysis, high-vacuum metal deposition, electron beam deposition, sputtering, ion beam sputtering, ion plating, ion-assisted deposition, and other methods.

When the substrate is made of an opaque material, the electrode formed on the substrate need not be transparent.

The light-emitting layer 3 is a layer that includes a light-emitting compound having the structure represented by formula (1) or (2), or a light-emitting polymer having the repeating unit represented by formula (3), in accordance with the present invention. The light-emitting layer 3 may be a high polymer film where the light-emitting compound or polymer is dispersed in a high polymer. The layer may also be a deposited film prepared by depositing the light-emitting compound on the transparent electrode 2.

Examples of the high polymer for the high polymer film are a polyvinyl carbazole, a poly (3-alkylthiophen) , a polyimide including an arylamide, a polyfluorene, a polyphenylene vinylene, a poly-α-methylstyrene, a copolymer of vinylcarbazole and α -methylstyrene. Of them, a polyvinyl carbazole is preferable.

The amount of the light-emitting compound or polymer included in the high polymer film is, typically 0. 01-2 weight%, preferably 0.05-0.5 weight%.

The thickness of the high polymer film ranges, typically between 30 nm and 500 nm, preferably between 100 nm and 300 nm. When the thickness is too small, the amount of the emitted light may be insufficient. On the other hand, when the thickness is too large, the voltage required to drive the illuminator or element may be too high, which is not desirable. Besides, the large thickness may reduce the flexibility of the film necessary to shape a planar, tubular, curved, or ring article.

The high polymer film may be formed through the application of a solution of the high polymer and the light-emitting compound or polymer of the invention dissolved in a suitable solvent. The application method is one selected from a spin cast method, a coating method, a dipping method, etc.

When the light-emitting layer 3 is a deposited film, the thickness of the film is generally 0.1-100 nm, although a preferable thickness is different depending on the structure of layers and other factors. When the thickness is too large or too small, it might cause the same problems as described above.

The light-emitting layer 3 may be made of the light-emitting polymer having the repeating unit represented by formula (3) *per se.* A typical example of forming the light-emitting polymer film on the transparent electrode 2 may be the application of a solution of the polymer dissolved in a suitable solvent onto the transparent electrode. The application method includes, for example, a spin cast method, a brush-coating method, etc. The formation of the light-emitting layer including this light-emitting polymer does not use the deposition method, which is often troublesome. Therefore the employment of the light-emitting polymer may sometimes be advantageous.

For the electrode layer 4 may be employed a material having a small work function. Examples of the material are elementary metals and metallic alloys, such as MgAg, aluminum alloy, metallic calcium, etc. A preferable electrode layer 4 is made of an alloy of aluminum and a small amount of lithium. This electrode may easily be formed on the surface of light-emitting layer 3, which, in turn, has been formed on substrate 1, by the technique of metal deposition.

When either of the deposition or the application is employed, a buffer layer should be inserted between each electrode and the light-emitting layer.

Materials for the buffer layer are, for example, an alkaline metal compound such as lithium fluoride, an alkaline earth metal compound such as magnesium fluoride, an oxide such as an aluminum oxide, and 4, 4'-biscarbazole biphenyl (Cz-TPD). Also, materials for forming the buffer layer between the cathode made of ITO, etc. and the organic layer are, for example, m-MTDATA (4,4',4"-tris(3-methylphenyl-phenylamino)triphenylamine), phthalocyanine, polyaniline, and polythiophene derivatives, and inorganic oxides such as molybdenum oxide, ruthenium oxide, vanadium oxide and lithium fluoride. When the materials are suitably selected, these buffer layers can lower the driving voltage of the organic EL element, which is the luminescent element, improve the quantum efficiency of luminescence, and achieve an increase in the luminance of the emitted light.

The second example of the luminescent element in accordance with this invention is shown in Figure 2. This figure is an illustration showing the sectional layer structure of an example of the luminescent element, which is a multi-layer organic EL element.

As shown in Figure 2, the luminescent element B comprises a substrate 1, and a transparent electrode 2, a hole-transporting layer 5, light-emitting sublayers 3a and 3b, an electron-transporting layer 6, and an electrode layer 4, the layers being laid on the substrate 1 one by one in this order.

The substrate 1, the transparent electrode 2 and the electrode layer 4 are the same as those explained for the luminescent element A in Figure 1.

The light-emitting layer of the luminescent element B comprises light-emitting sublayers 3a and 3b. The light-emitting sublayer 3a is a film including the light-emitting compound or polymer of this invention. The light-emitting sublayer 3b is a DPVBi layer, which functions as a host material.

Examples of the hole-transporting substance included in the hole-transporting layer 5 are a triphenylamine compound such as N,N'-diphenyl-N,N'-di(m-tolyl)-benzidine (TPD) and α -NPD, a hydrazon compound, a stilbene compound, a heterocyclic compound, a π electron star burst positive hole transporting substance, etc.

Examples of the electron-transporting substance included in the electron-transporting layer 6 are an oxadiazole derivative such as 2-(4-tert-butylphenyl)-5-(4-biphenylyl)-1,3,4-oxadiazole and 2,5-bis(1-naphthyl)-1,3,4-oxadiazole (END), and 2,5-bis(5'-tert-butyl-2'-benzoxazolyl) thiophene. Also, a metal complex material such as quinolinol aluminum complex (Alq3), benzoquinolinol beryllium complex (Bebq2) may be used suitably.

The electron-transporting layer 6 of the luminescent element B shown in Figure 2 includes Alq3 as electron-transporting substance.

The thickness of each layer is the same as that in a known multi-layer organic EL element.

The luminescent element B in Figure 2 functions and emits light in the same ways as the luminescent element A in Figure 1. Therefore, the luminescent element B has the same uses as the luminescent element A.

The third example of the luminescent element of this invention is shown in Figure 3. This figure is an illustration showing the sectional layer structure of an example of the luminescent element, which is a multi-layer organic EL element.

The luminescent element C shown in Figure 3 comprises a substrate 1, and a transparent electrode 2, a hole-transporting layer 5, a light-emitting layer 3, an electron-transporting layer 8, and an electrode layer 4, wherein the transparent electrode and the layers are laid on substrate 1 one by one in this order.

The luminescent element C functions in the same way as the luminescent element B.

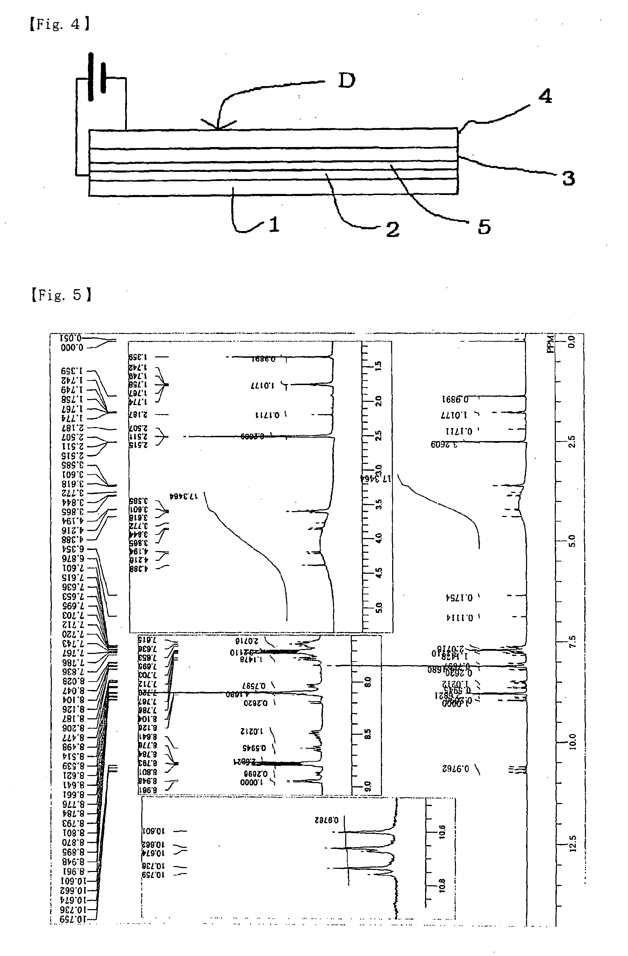

Another example of the luminescent element of this invention is shown in Figure 4. The luminescent element D comprises a substrate 1, and a transparent electrode 2, a hole-transporting layer 5, a light-emitting layer 3, and an electrode layer 4 wherein the transparent electrode and the layers are laid on the substrate 1 one by one in this order.

An example of the luminescent elements, other than those shown in Figures 1-4, is a two-layer low molecular weight organic luminescent element having a hole-transporting layer that includes a hole-transporting substance and an electron-transporting luminescent layer that includes a light-emitting compound or polymer of the invention laid on the hole-transporting layer, these layers being sandwiched between a cathode, which is the transparent electrode formed on the substrate, and an anode, which is the electrode layer. A specific example of this embodiment is a two-layer pigment-injected luminescent element comprising a hole-transporting layer and a light-emitting layer that includes a host pigment and a light-emitting compound or polymer of this invention as a guest pigment, wherein the light-emitting layer is laid on the hole-transporting layer and these layers are sandwiched between the cathode and the anode. Another example is a two-layer organic luminescent element comprising a hole-transporting layer that includes a hole-transporting substance and an electron-transporting luminescent layer that is prepared through a co-deposition of a light-emitting compound or polymer of the invention and an electron-transporting substance, the latter layer being laid on the former, and these two layers being sandwiched between the cathode and the anode. A specific example of the second embodiment is a two-layer pigment-injected luminescent element comprising a hole-transporting layer and an electron-transporting luminescent layer that includes a host pigment and a light-emitting compound or polymer of this invention as a guest pigment, wherein the luminescent layer is laid on the hole-transporting layer and these layers are sandwiched between the cathode and the anode. A further example is a three-layer organic luminescent element comprising a hole-transporting layer, a luminescent layer including a light-emitting compound or polymer of this invention that is laid on the hole-transporting layer, and an electron-transporting layer that is laid on the luminescent layer, these layers being sandwiched between the cathode and the anode.

The electron-transporting luminescent layer, typically, comprises 50-80 weight% of polyvinylcarbazole, which is abbreviated to PVK, 5-40 weight% of an electrode-transporting luminescent agent, and 0.01-20 weight% of the light-emitting compound or polymer of this invention. The electron-transporting luminescent layer of this composition is capable of emitting light at a high luminance. The color of the light emitted by the luminescent element according to the present invention is typically white.

Also, it is preferable, if the light-emitting layer includes, as a sensitizing agent, rubrene, especially both of rubrene and A1q3.

An illuminator utilizing the compound or polymer of this invention may generally be used as an organic EL element which is driven, generally, by direct current, and also by pulses and alternating current.

### Examples

### (Example 1) Synthesis of Light-emitting Compound and Polymer

In a 2L three-necked flask were placed 20 g of the compound represented by formula (4) below, 14.6 g of the compound represented by formula (5), 7.4 ml of pyridine, and 500 ml of tetrahydrofuran. The flask containing the mixture was placed in a silicone oil bath and the mixture was heated to 70°C. The reaction was allowed to continue for 2.5 hours. After the termination of the reaction, the solution in the flask was cooled naturally. The solution was then concentrated and filtered. The solids obtained by the filtration were dried. 17 g of a solid matter was obtained.

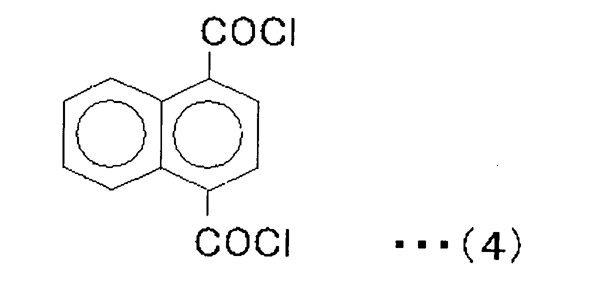

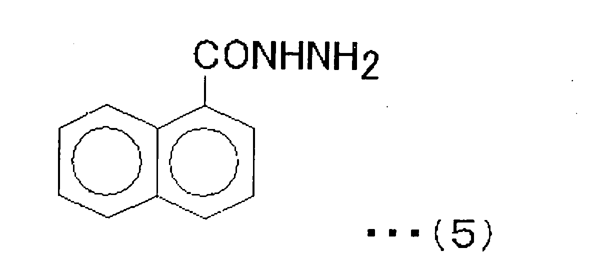

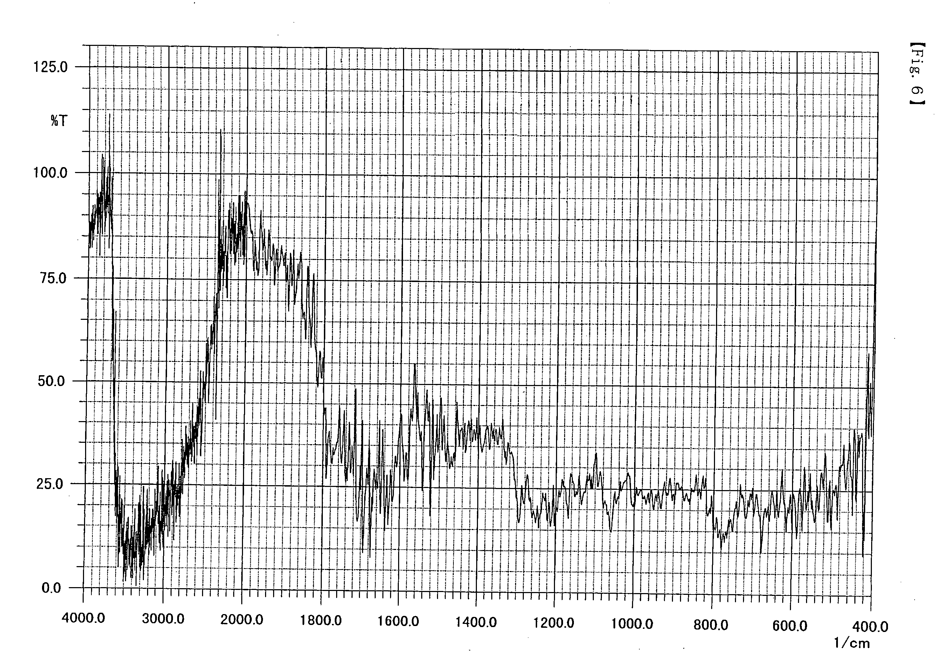

An NMR spectrum chart and an IR spectrum chart of the solid matter are respectively shown in Figure 5 and Figure 6. This solid matter was identified as a compound having the structure represented by formula (6) below.

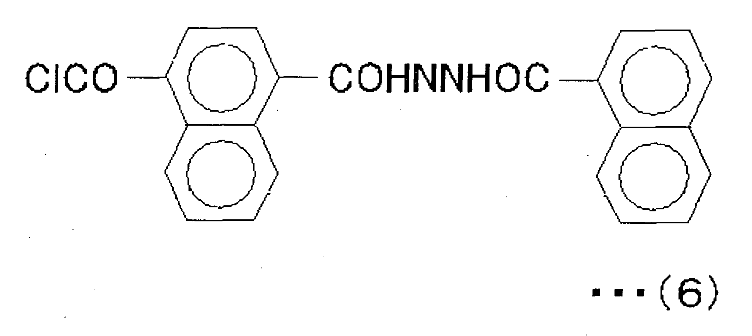

Next, in a 2L three-necked flask, 17 g of the compound shown by formula (6), 13.4 ml of hydrazine, 10 ml of pyridine, and 800 ml of tetrahydrofuran were placed. The flask containing the mixture was placed in a silicone oil bath and the mixture was heated to 55°C. The reaction was allowed to continue for 2 hours. After the termination of the reaction, the resultant solution was cooled naturally. After the cooling, the solution was filtered. Then, 700 ml of chloroform was added to the filtrate, and the resultant mixture was extracted. The extract was washed with water, and the moisture included in the extract was removed with sodium sulfate. The dried extract was filtered and dried up. 4.6 g of a solid matter was obtained.

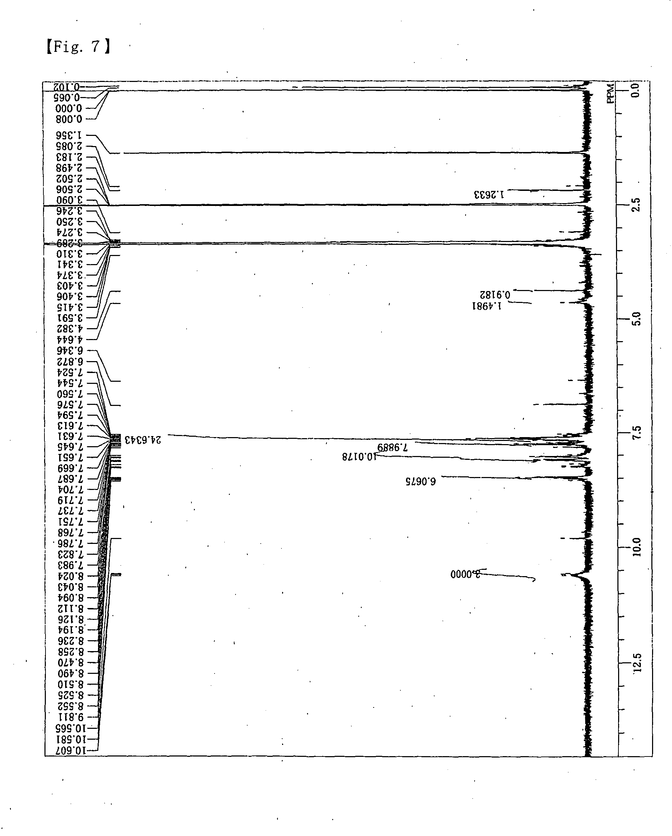

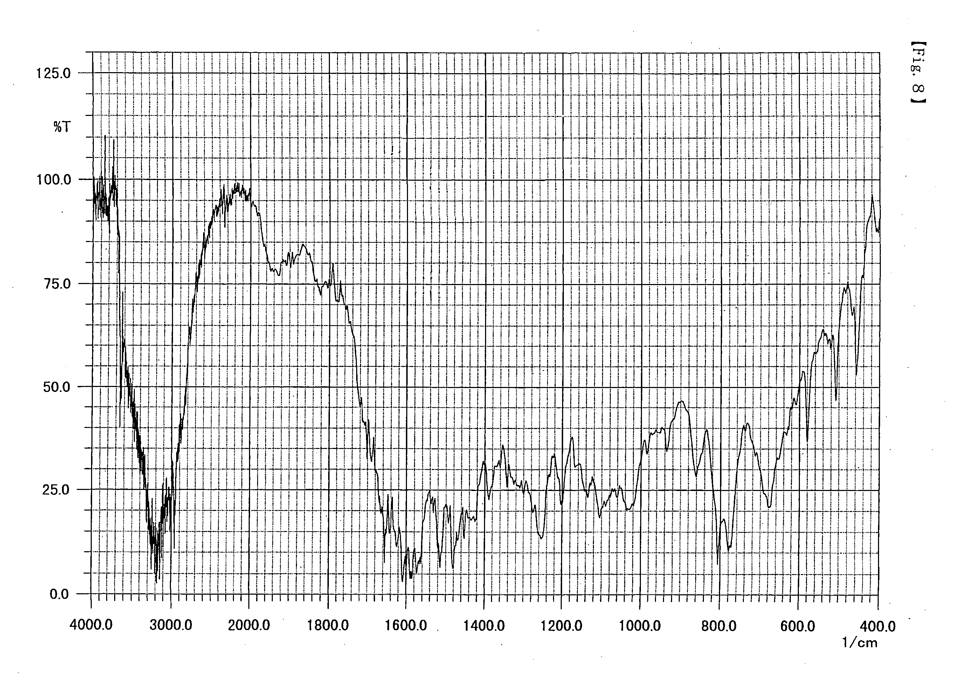

An NMR spectrum chart and an IR spectrum chart of the solid matter are respectively shown in Figure 7 and Figure 8. The solid matter was identified as a compound having the structure represented by formula (7).

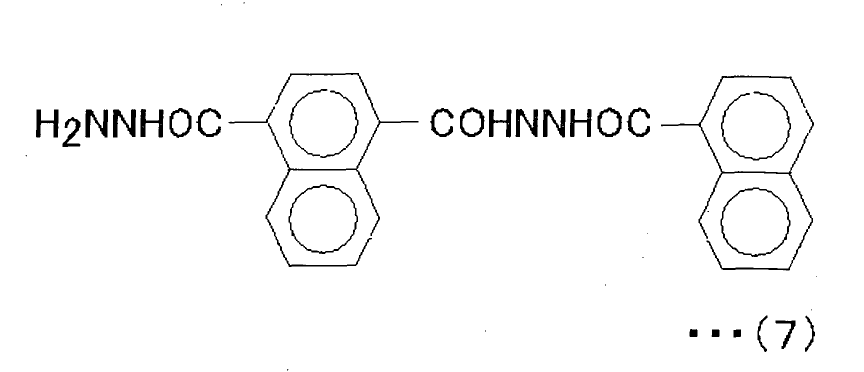

Then, in a 2L three-necked flask were placed 4.6 g of the compound represented by formula (7), 3.27 g of the compound represented by formula (8) below, 1.5 g of pyridine, and 600 ml of tetrahydrofuran. The flask containing the solution was placed in a silicone oil bath and the solution was heated to 60°C. The reaction was allowed to continue for 10 hours. After the termination of the reaction, the solution in the flask was cooled naturally. The solution was then concentrated and filtered. The filtrate was washed with water, and then with methanol. The washed filtrate was dried up to a solid matter of 1.15 g.

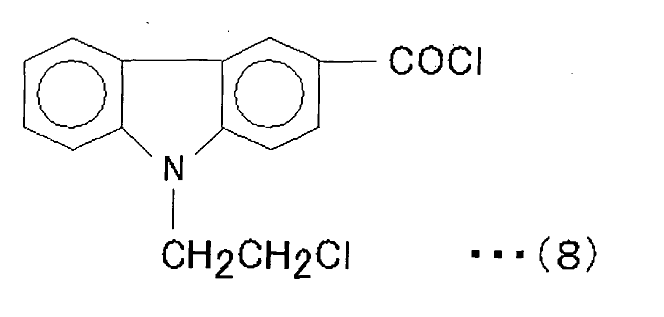

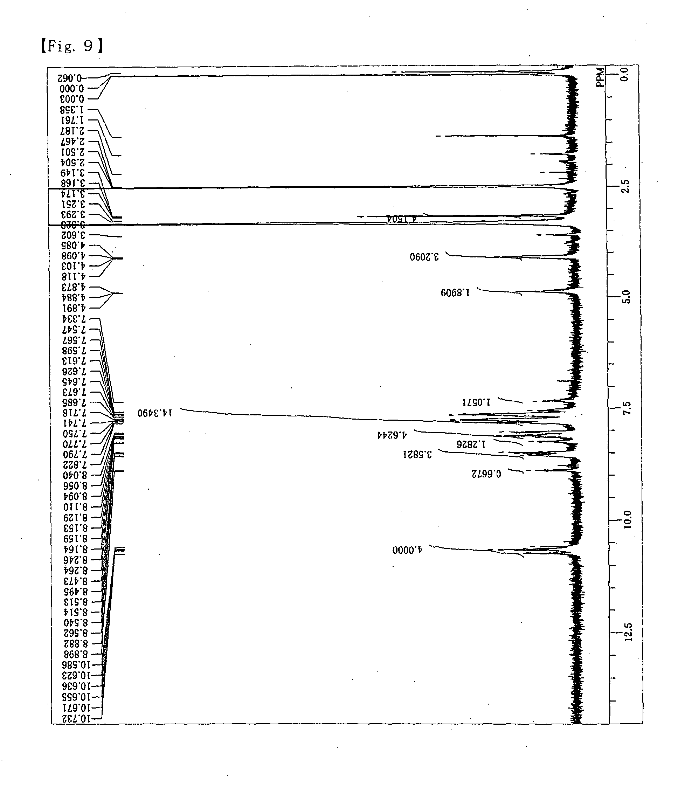

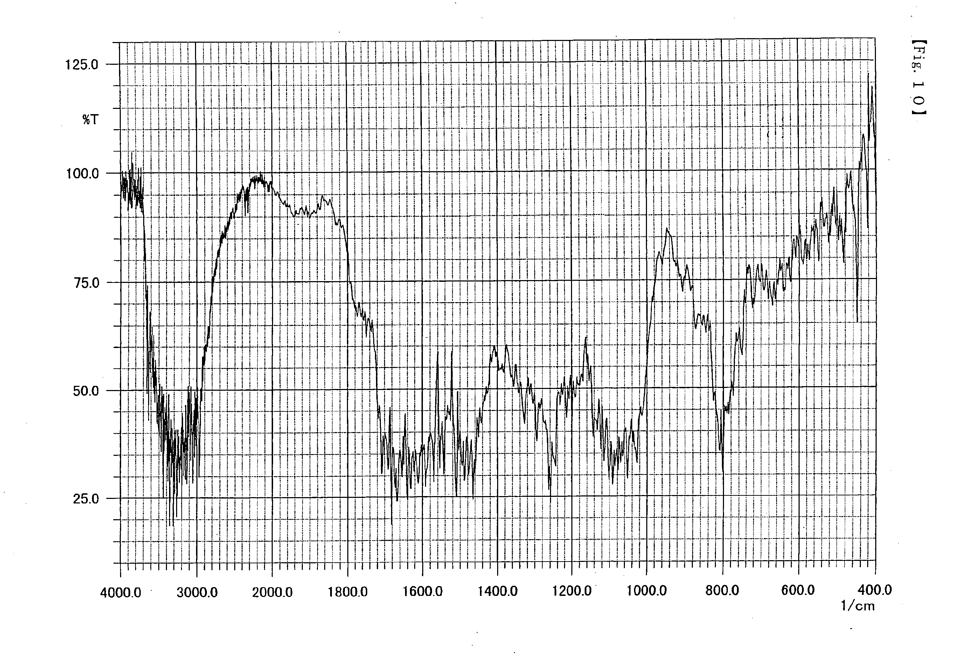

An NMR spectrum chart and an IR spectrum chart of the solid matter are respectively shown in Figure 9 and Figure 10. The solid matter was identified as a compound having the structure represented by formula (9).

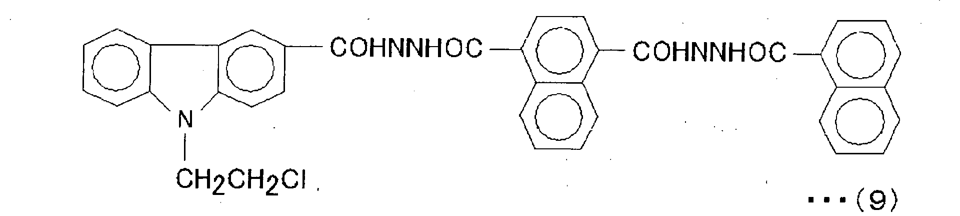

Then, in a 500 ml pear-shaped flask were placed 1.15 g of the compound represented by formula (9), 150 ml of 1,4-dioxane, and 75 ml of phosphoryl chloride. The flask containing the solution was placed in a silicone oil bath and the solution was heated to 110°C. The reaction was allowed to continue for 15.5 hours. After the termination of the reaction, the solution was cooled naturally. The solution was then filtered, and the filtrate was washed with water and methanol in this order. The washed filtrate was dried up to a solid matter of 0.65 g.

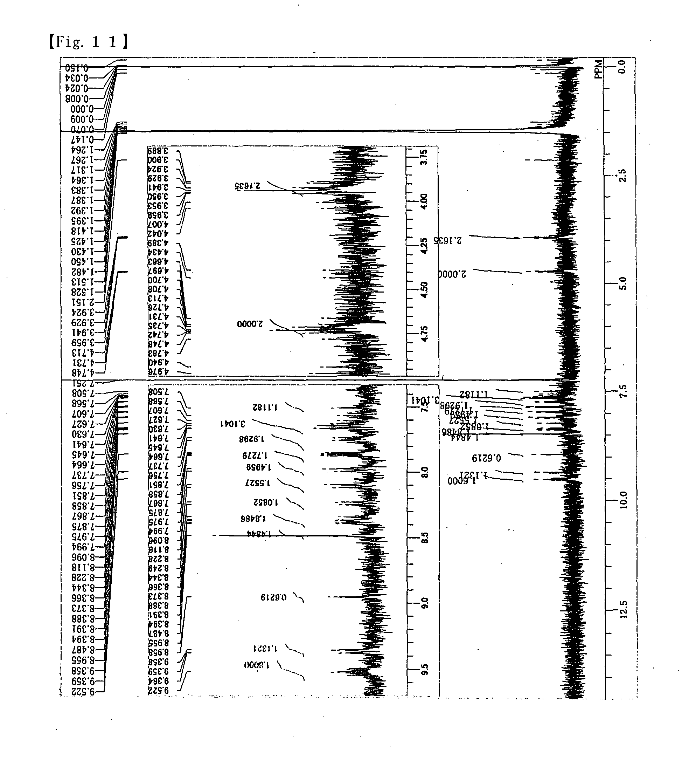

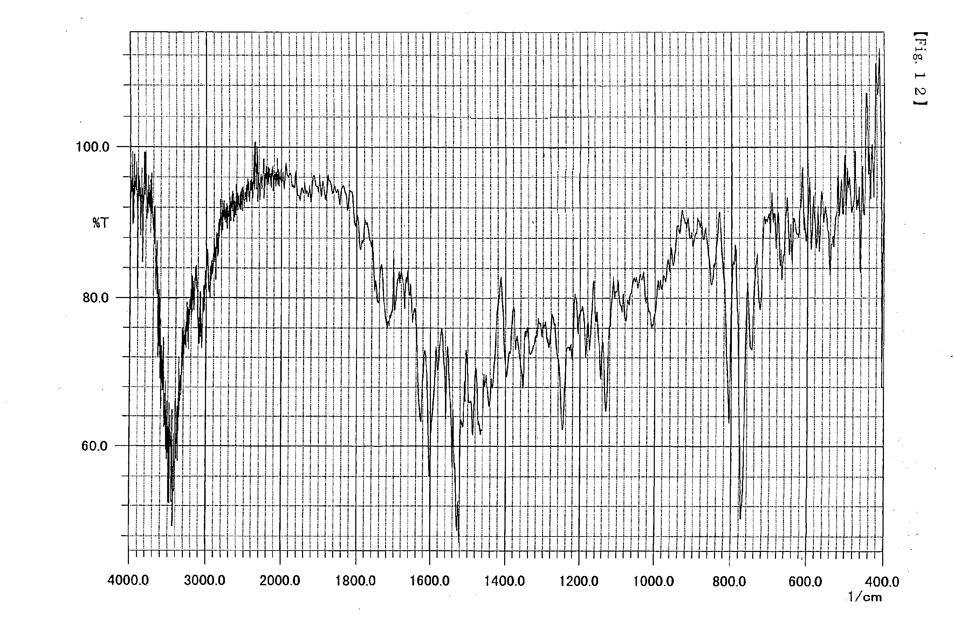

An NMR spectrum chart and an IR spectrum chart of the solid matter are respectively shown in Figure 11 and Figure 12. From these data, the solid matter was identified as a compound having the structure represented by formula (10).

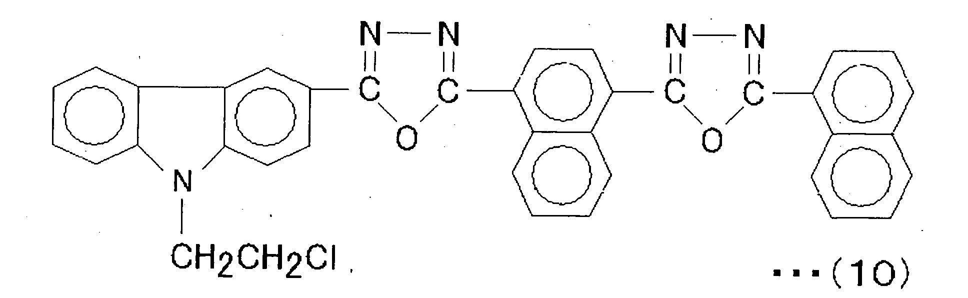

In a 500 ml pear-shaped flask were placed 0.65 g of the compound represented by formula (10), 0.7 g of potassium hydroxide, 100 ml of 1,4-dioxane, and 50 ml of ethanol. The flask containing the solution was placed in a silicone oil bath and the solution was heated to 100°C. The reaction was allowed to continue for 16.5 hours. After the termination of the reaction, the solution was cooled naturally. The solution was concentrated and filtered. The filtrate was washed with water and then with methanol. The washed filtrate was dried up to a solid matter of 0.15 g.

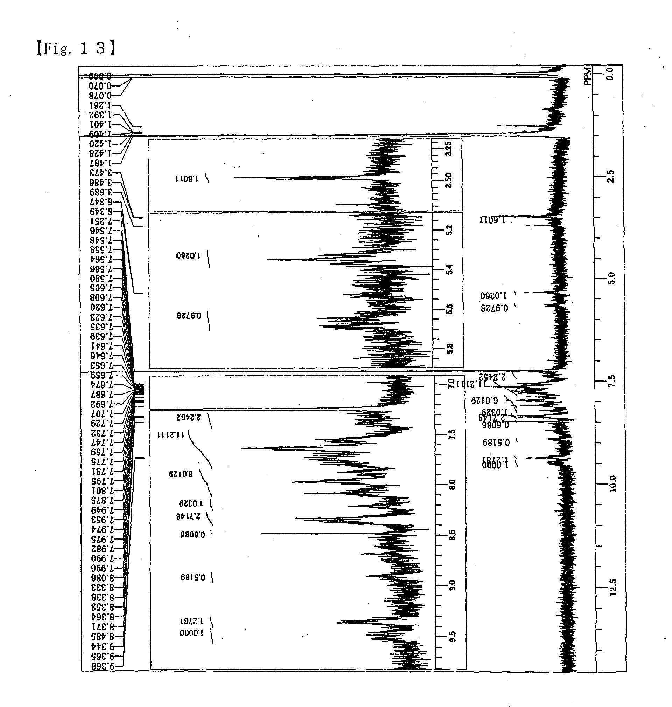

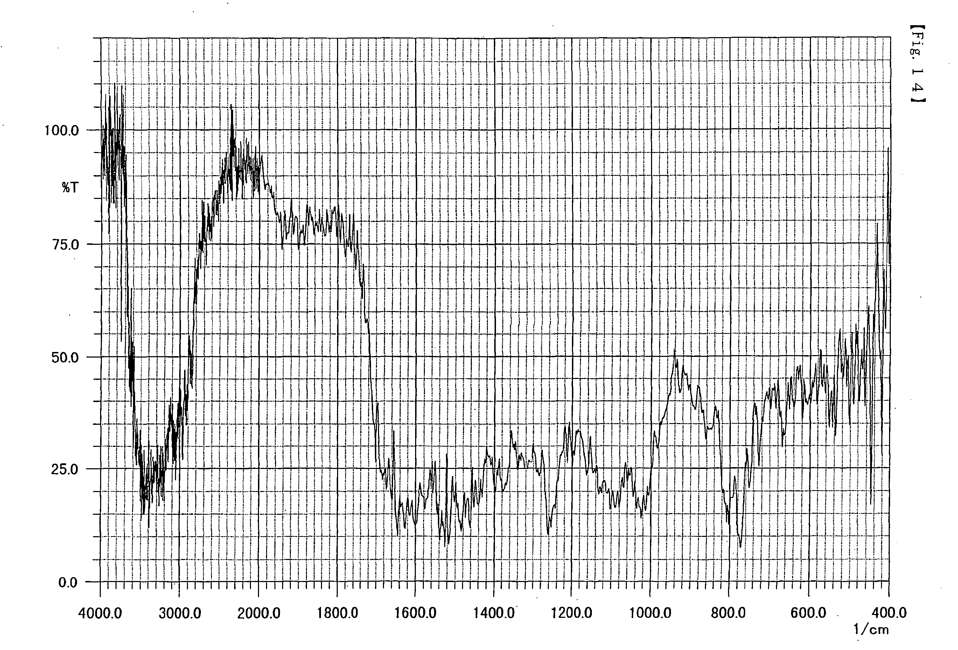

An NMR spectrum chart and an IR spectrum chart of the solid matter are respectively shown in Figure 13 and Figure 14. From these data, the solid matter was identified as a compound having the structure represented by formula (11).

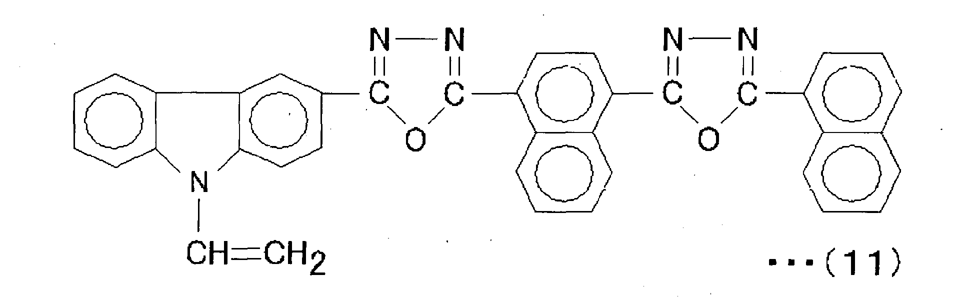

A fluorescence spectrum chart of this compound is shown in Figure 15. This chart shows that the compound obtained emits white light.

Next, 0.1 g of the compound represented by formula (11), 0.54 mg of AIBN, 50 ml of chlorobenzene, and 5 ml of DMF were placed in a polymerizing tube. The solution in the tube was heated in a silicone oil bath for 48 hours at 145°C. Then, the solution in the tube was concentrated, cooled naturally, and filtered. The filtrate was dried, and 0.1 g of a solid matter was obtained.

### (Example 2) Synthesis of Light-emitting Polymer

A 1L pear-shaped flask was charged with 15 g of 9,9'-dimethylflorene-2,7-dicarboxylic acid and 200 ml of thionyl chloride. The solution in the pear-shaped flask was heated in a silicone oil bath to 80°C and allowed to react for 3 hours. After the completion of the reaction, the solution in the flask was cooled with ice. Then, thionyl chloride was distilled away, which resulted in the precipitation of solids. The solids were dried, and 31 g of a white solid matter was obtained.

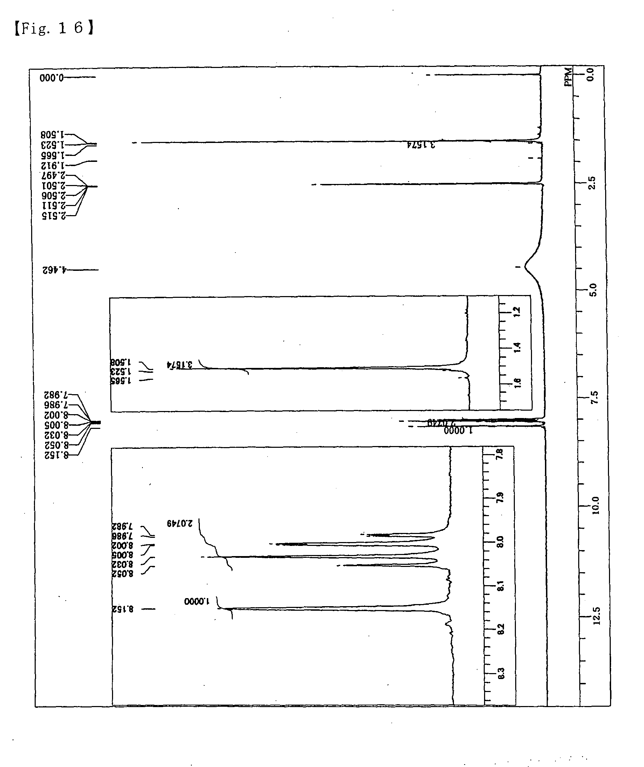

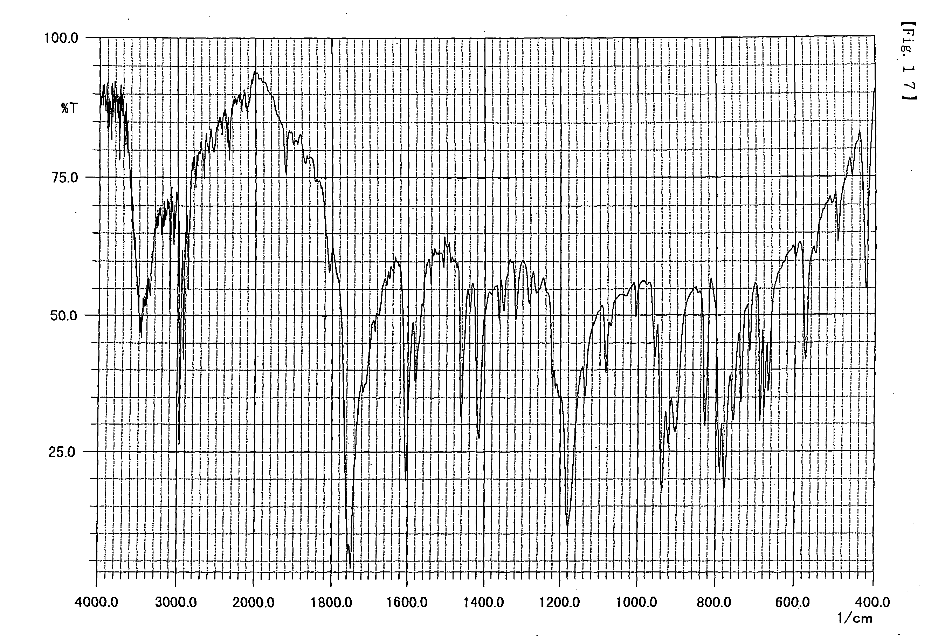

An NMR spectrum chart and an IR spectrum chart of the obtained solid matter are respectively shown in Figure 16 and Figure 17. The obtained was identified as an acid chloride compound having the structure represented by formula (12).

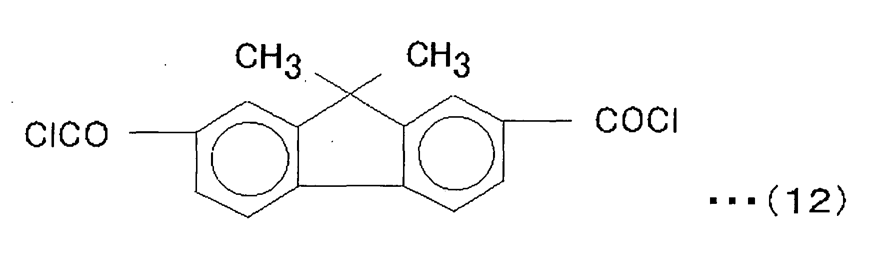

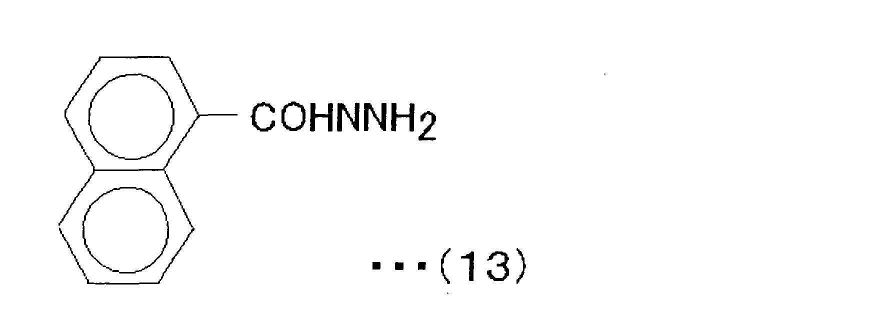

Then, in a 500 ml three-necked flask were placed 10 g of the compound represented by formula (13) above, 5 g of pyridine, and 300 ml of tetrahydrofuran. A solution was made by dissolving the solid matter represented by formula (12) in 500 ml of tetrahydrofuran. The entire amount of the solution was dripped into the solution in the three-necked flask, while the solution in the flask was being cooled with ice. After the completion of dripping, the solution in the three-necked flask was heated in a silicone oil bath to 60°C and allowed to react for 16 hours. After the completion of the reaction, the solution was cooled with ice, and filtered. The precipitated solids were collected, washed with water, and dried. The dried solids were further dried up, and 18.75 g of a brown solid matter was obtained.

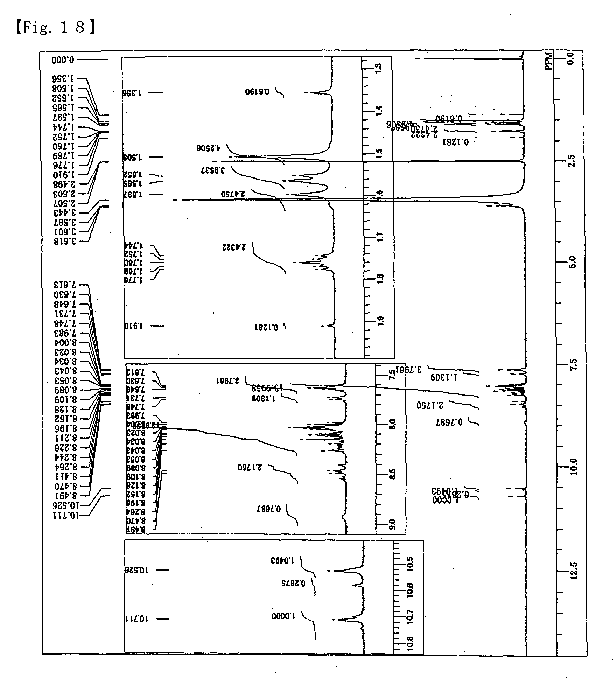

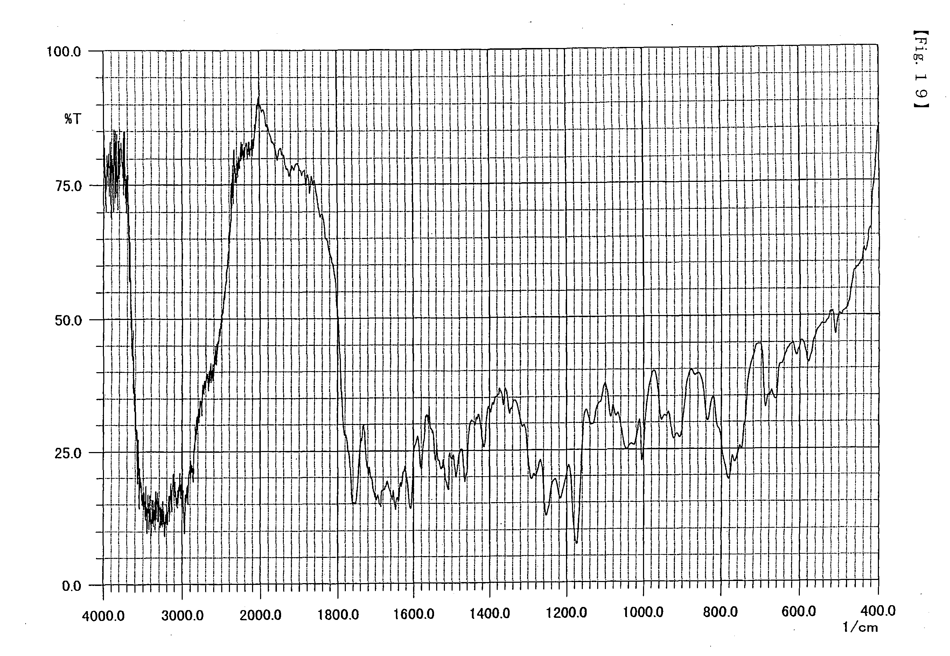

An NMR spectrum chart and an IR spectrum chart of the obtained solid matter are respectively shown in Figure 18 and Figure 19. The obtained was identified as a hydrazide having the structure represented by formula (14).

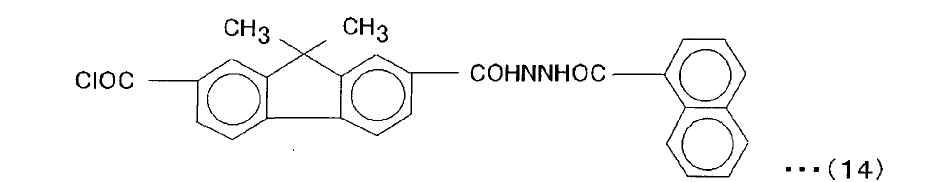

Next, in a 500 ml three-necked flask, 18.75 g of the compound shown by formula (14), 9.6 ml of pyridine, 13 ml of hydrazine, and 700 ml of tetrahydrofuran were placed. The flask containing the solution was placed in a silicone oil bath and the solution was heated to 60°C. The reaction was allowed to continue for 12 hours. After the termination of the reaction, the resultant solution was cooled with ice. Then, the cooled was filtered, and the precipitated solids were collected. The solids were washed with water and dried. The dried solids were further dried up. As a result, 4.42 g of a brown solid matter was obtained.

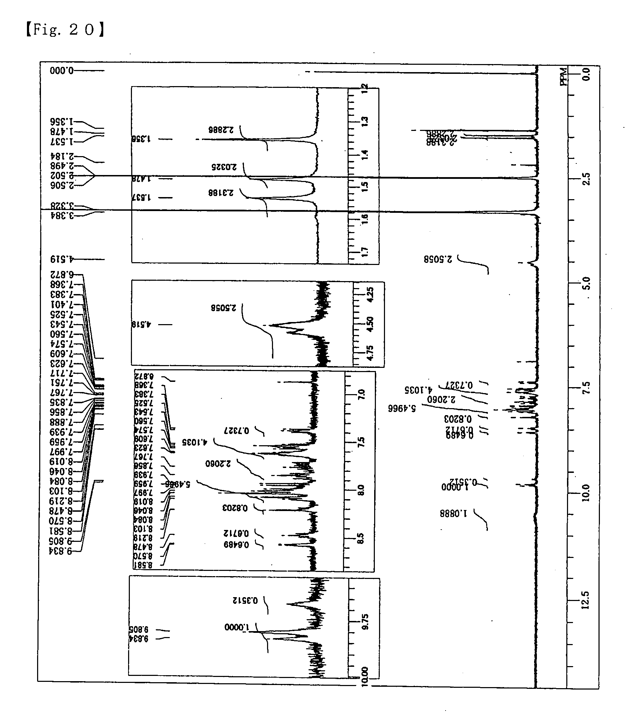

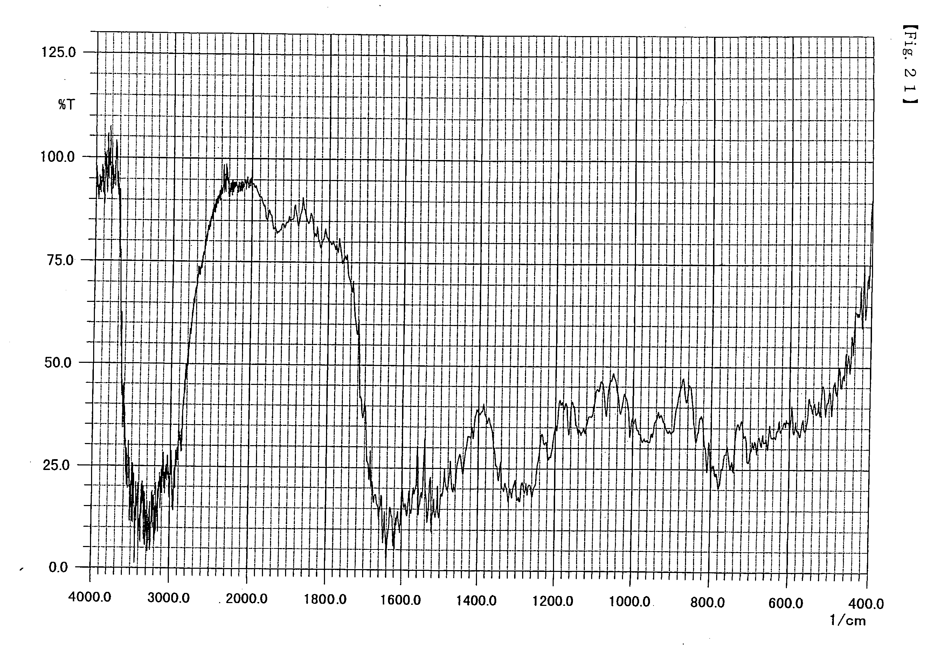

An NMR spectrum chart and an IR spectrum chart of the solid matter are respectively shown in Figure 20 and Figure 21. The solid matter was identified as a compound having the structure represented by formula (15).

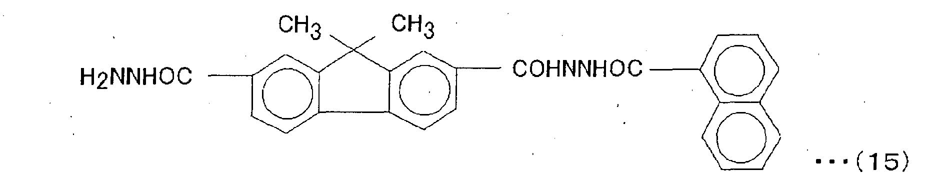

Next, in a 500 ml three-necked flask were placed 2.44 g of the compound represented by formula (15) above, 0.5 g of pyridine, and 100 ml of tetrahydrofuran. A solution was made by dissolving 1.5 g of the compound represented by formula (8), which was also used in Example 1, in 500 ml of tetrahydrofuran. The entire amount of the solution was dripped into the solution in the three-necked flask, while the solution in the flask was being cooled with ice. After the completion of dripping, the solution in the three-necked flask was heated in a silicone oil bath to 70°C and allowed to react for 1.5 hours. After the completion of the reaction, the solution was cooled with ice, and filtered. The precipitated solids were collected, washed with water, and dried. The dried solids were further dried up, and 1.27 g of a yellow solid matter was obtained.

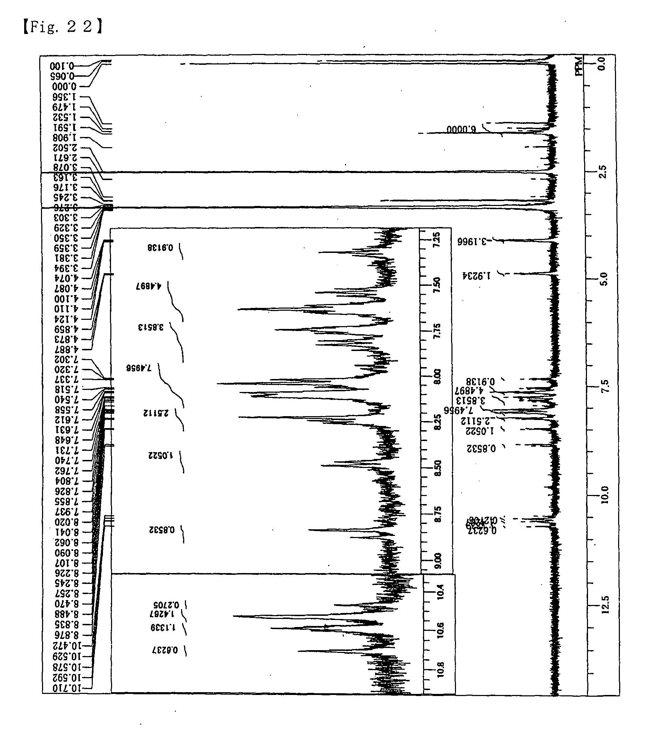

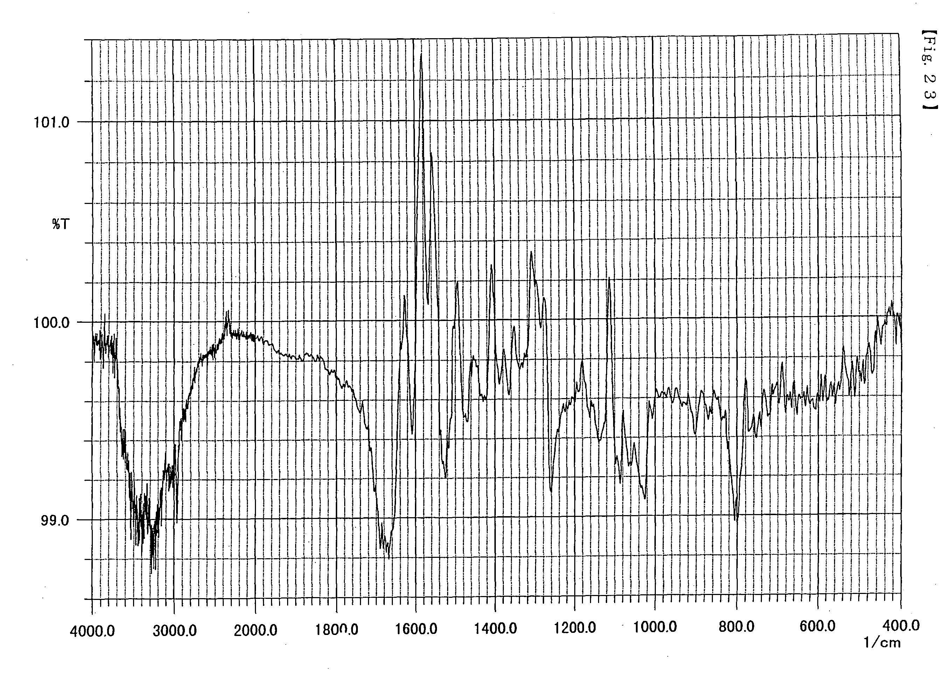

An NMR spectrum chart and an IR spectrum chart of the obtained solid matter are respectively shown in Figure 22 and Figure 23. The obtained was identified as a hydrazide having the structure represented by formula (16).

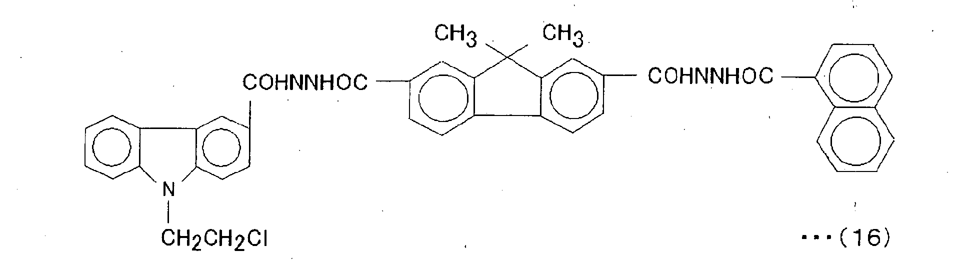

In a 500 ml three-necked flask were placed 1.27 g of the compound represented by formula (16), 50 ml of 1,4-dioxane, and 30 ml of phosphoryl chloride. The flask containing the solution was placed in a silicone oil bath and the solution was heated to 115°C. The reaction was allowed to continue for 6 hours. After the termination of the reaction, the contents in the flask were filtered, and solids were removed. The filtrate was washed with water and methanol in this order. Then, the washed filtrate was dried with sodium sulfate, and 0.5 g of a brown solid matter was obtained.

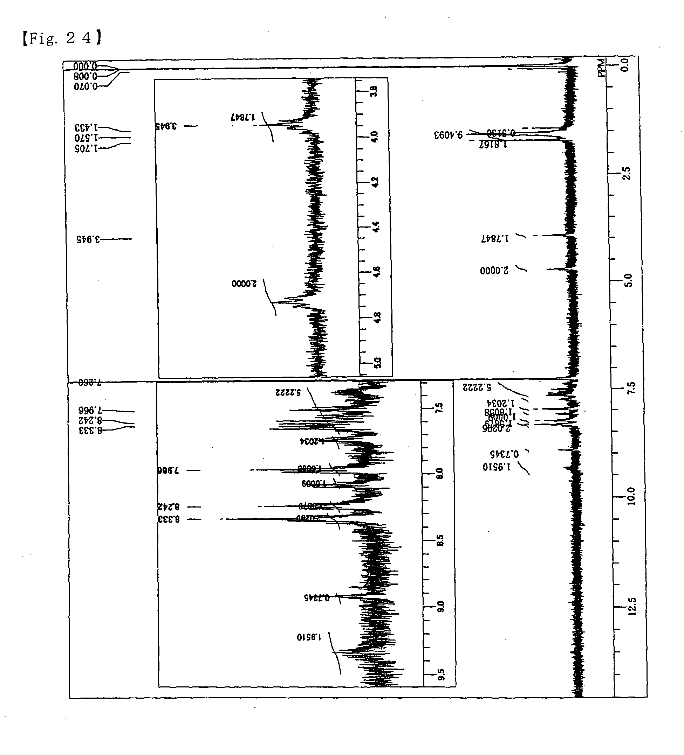

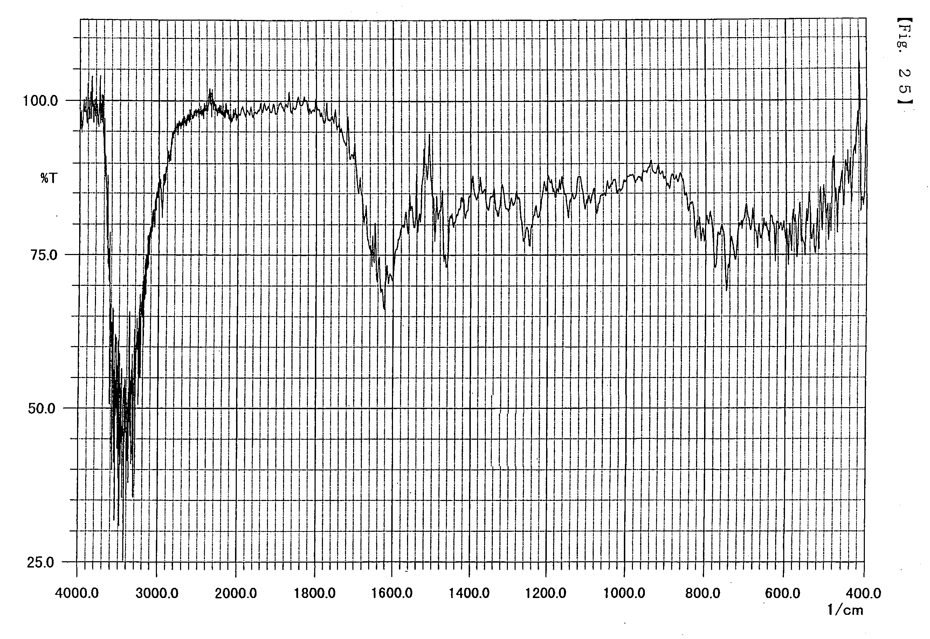

An NMR spectrum chart and an IR spectrum chart of the solid matter are respectively shown in Figure 24 and Figure 25. From these data, the solid matter was identified as a compound having the structure represented by formula (17).

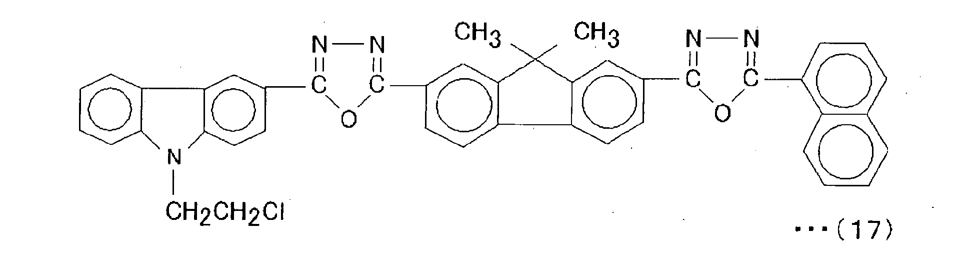

In a 500 ml pear-shaped flask were placed 0.36 g of the compound represented by formula (17), 0.77 g of potassium hydroxide, and 100 ml of 1,4-dioxane. The flask containing the solution was placed in a silicone oil bath and the solution was heated to 100°C. The reaction was allowed to continue for 3 hours. After the termination of the reaction, the solution was concentrated. The concentrate was extracted with 300 ml of chloroform. Moisture in the extract was removed with 50 g of anhydrous sodium sulfate. The dehydrated extract was concentrated, and the concentrate was dried up. 0.07 g of a reddish brown solid matter was obtained.

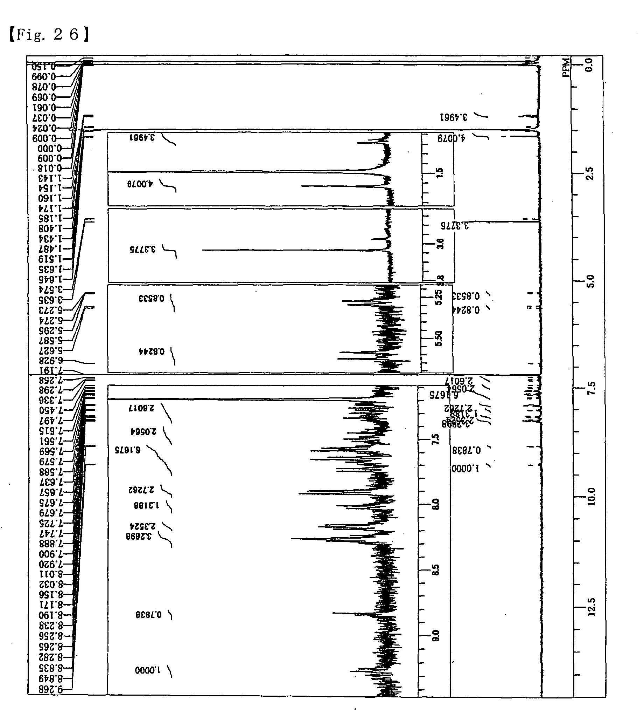

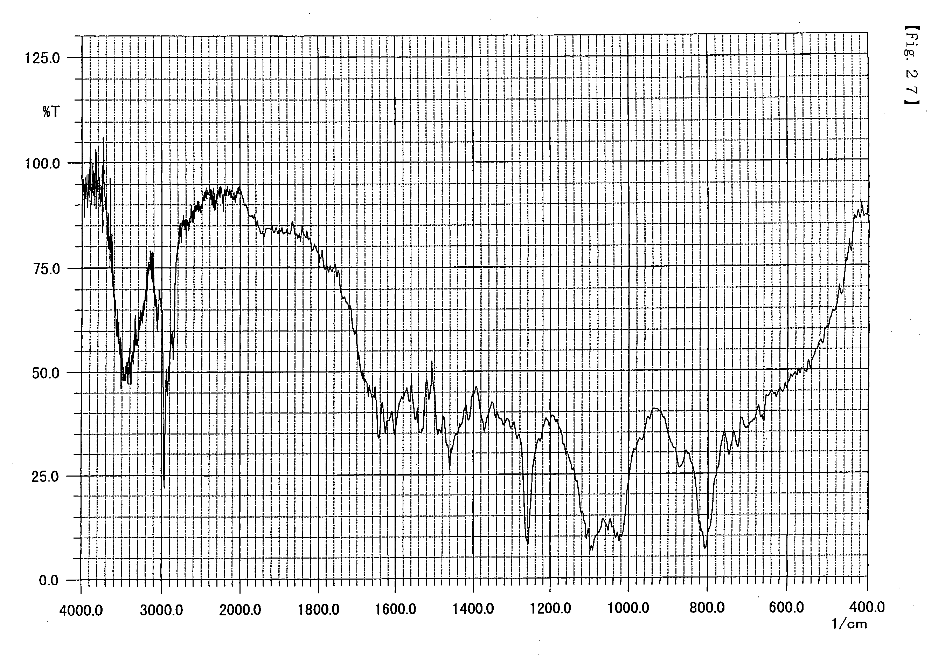

An NMR spectrum chart and an IR spectrum chart of the solid matter are respectively shown in Figure 26 and Figure 27. From these data, the solid matter was identified as a vinyl compound having the structure represented by formula (18).

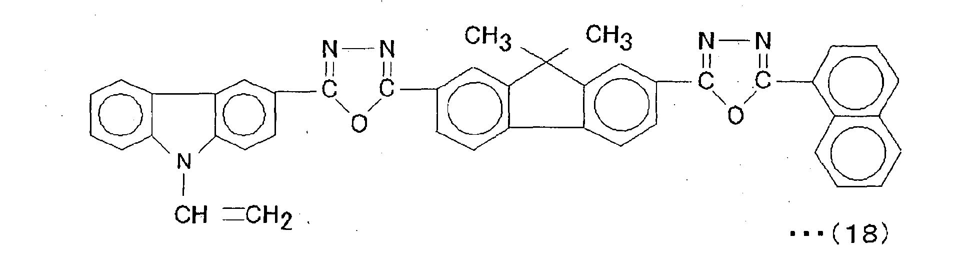

0.07 g of the vinyl compound represented by formula (18), 5 ml of ortho-dichlorobenzene, and 5 ml of DMF were placed in a polymerizing tube. The solution in the tube was heated in a silicone oil bath for 50 hours at 145°C. Then, the solution in the tube was concentrated, and filtered. The concentrate was dried up, and 0.051 g of an ash-colored solid matter was obtained.

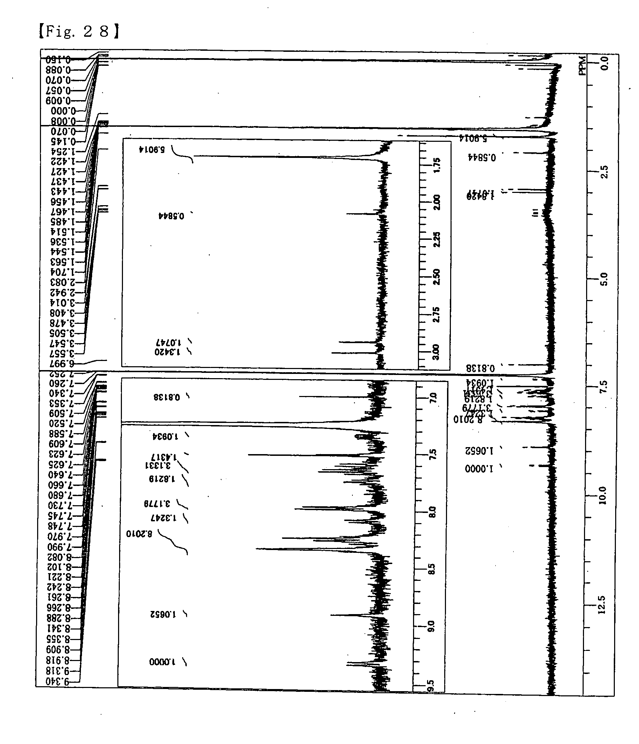

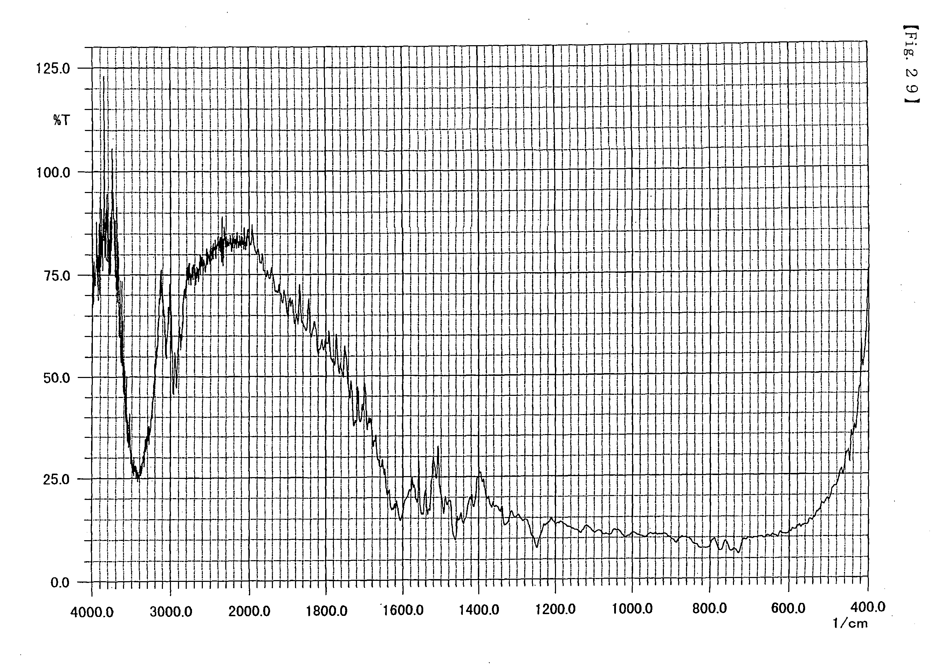

An NMR spectrum chart and an IR spectrum chart of the solid matter are respectively shown in Figure 28 and Figure 29. Fromthesedata, thesolidmatterwasidentifiedasapolymer having the repeating unit represented by formula (19).

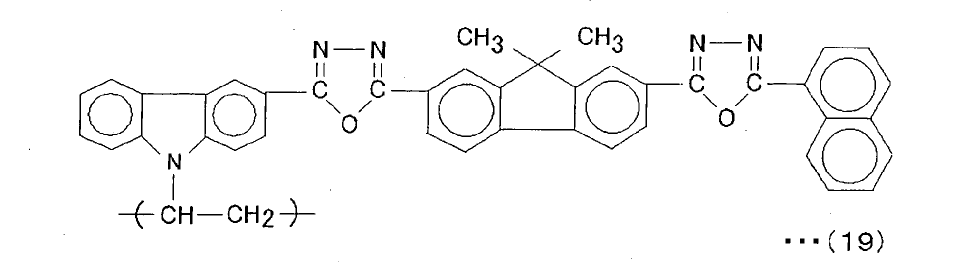

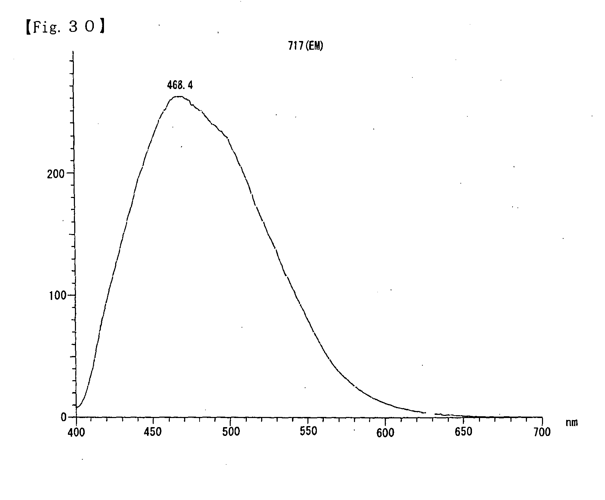

A fluorescence spectrum chart of this compound is shown in Figure 30. This chart shows that the polymer obtained emits white light.

### (Example 3) Synthesis of Light-emitting Compound and Polymer

A 2L three-necked flask was charged with 9 g of 9,9'-dimethylflorene-2,7-dicarboxylic acid, 450 ml of 1,4-dioxane, 3 g of pyridine, and 150 ml of thionyl chloride. The solution in the three-necked flask was heated in a silicone oil bath and allowed to react for 7 hours. After the completion of the reaction, the solution in the flask was cooled naturally. Then, the cooled solution was concentrated and filtered. 9.15 g of light pink crystals were obtained.

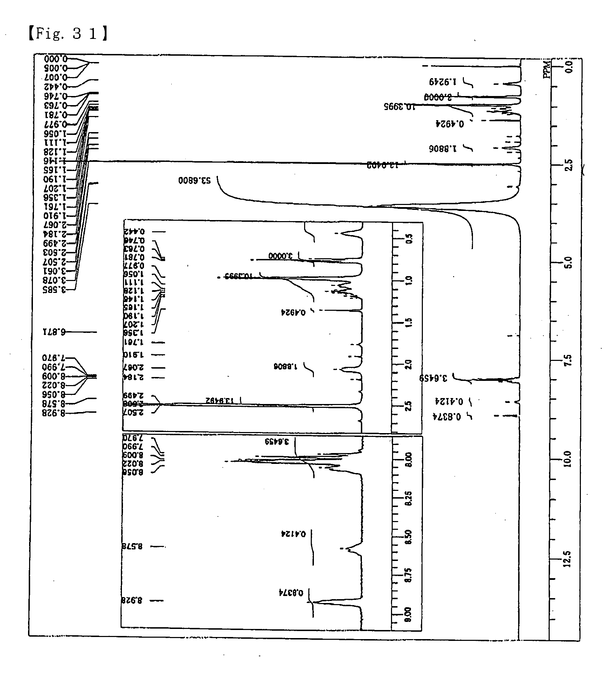

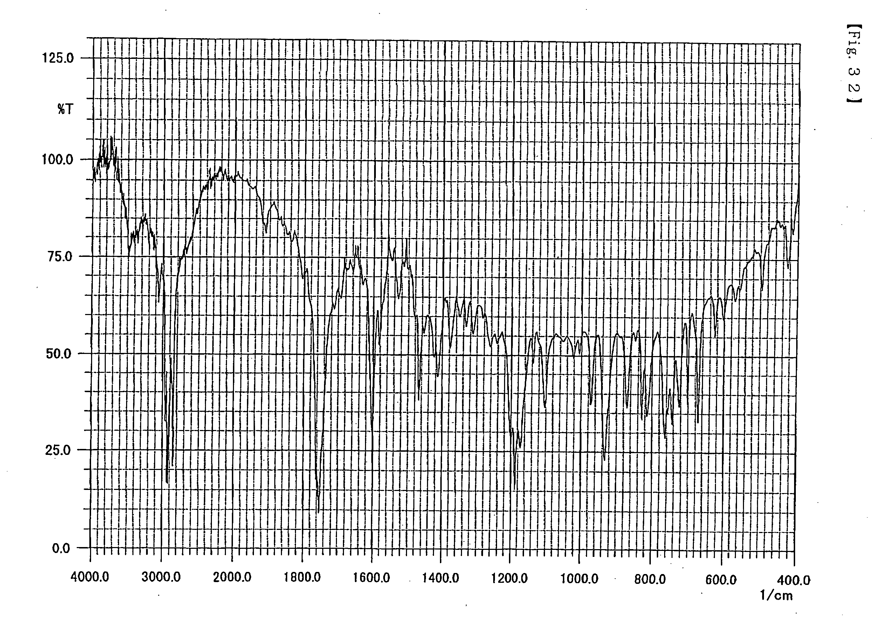

An NMR spectrum chart and an IR spectrum chart of the crystals are respectively shown in Figure 31 and Figure 32. The obtained was identified as a compound having the structure represented by formula (20).

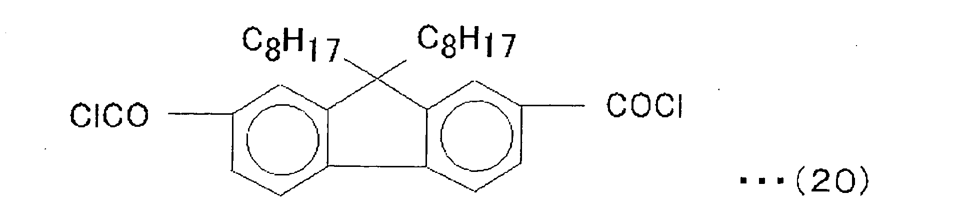

Then, in a 2L three-necked flask were placed 9.15 g of the acid chloride compound represented by formula (20), 3.31 g of the compound represented by formula (13) above, 1.69 g of pyridine, and 300 ml of tetrahydrofuran. The solution in the three-necked flask was heated in a silicone oil bath to 70°C and allowed to react for 1.5 hours. After the completion of the reaction, the resultant solution was cooled naturally, concentrated, and filtered. 12 g of a solid matter was obtained.

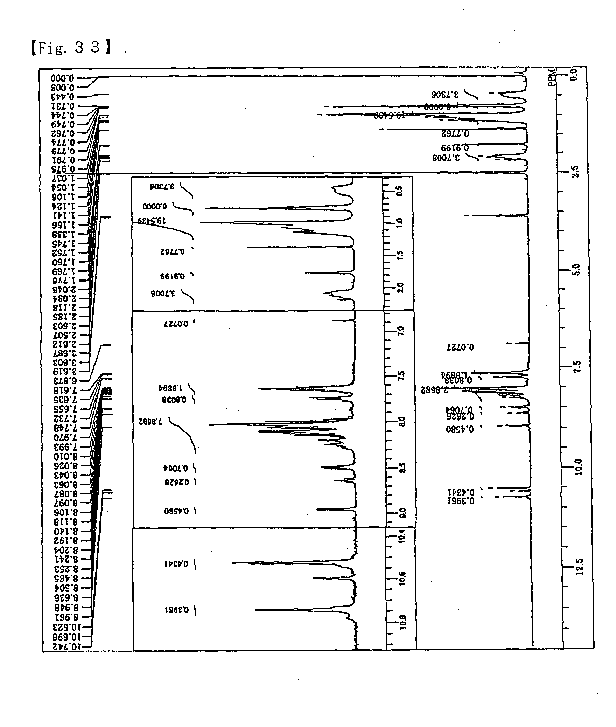

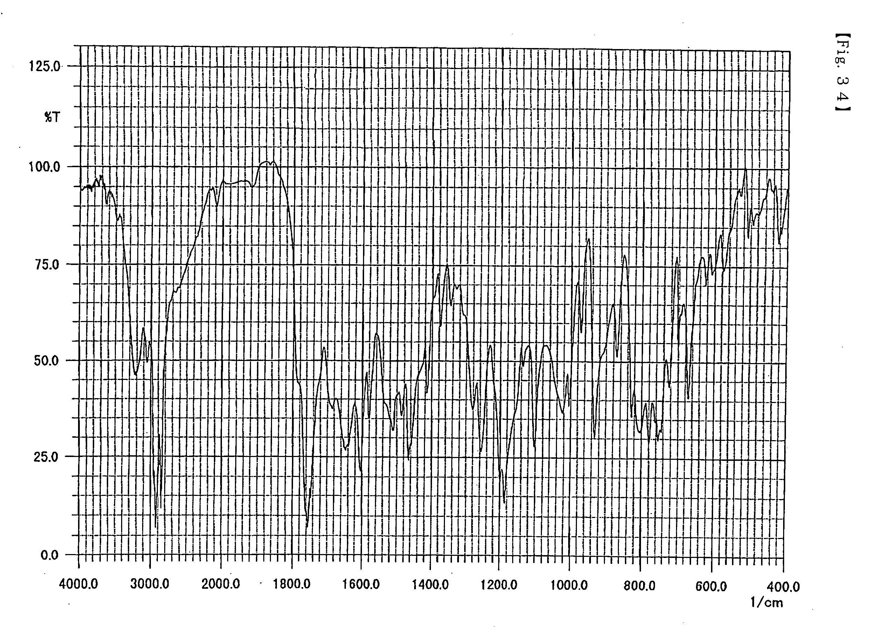

An NMR spectrum chart and an IR spectrum chart of the obtained solid matter are respectively shown in Figure 33 and Figure 34. The obtained was identified as a hydrazide having the structure represented by formula (21).

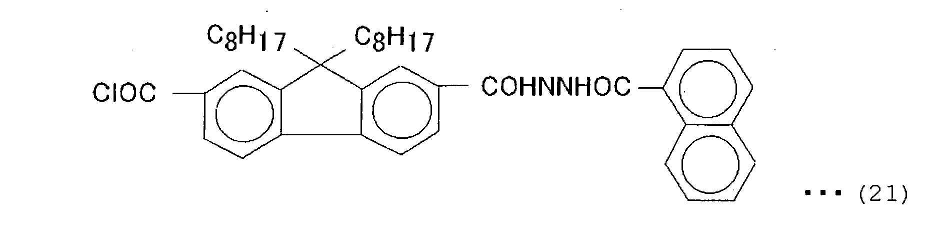

Next, in a 1L three-necked flask, 12 g of the compound shown by formula (21), 4.27 ml of pyridine, 5.8 ml of hydrazine, and 400 ml of tetrahydrofuran were placed. The flask containing the solution was placed in a silicone oil bath and the solution was heated to 70°C. The reaction was allowed to continue for 4. 5 hours. After the termination of the reaction, the resultant solution in the flask was extracted with chloroform. The extract was distilled with an evaporator and dried using a vacuum pump. As a result, 9.37 g of brown powder was obtained.

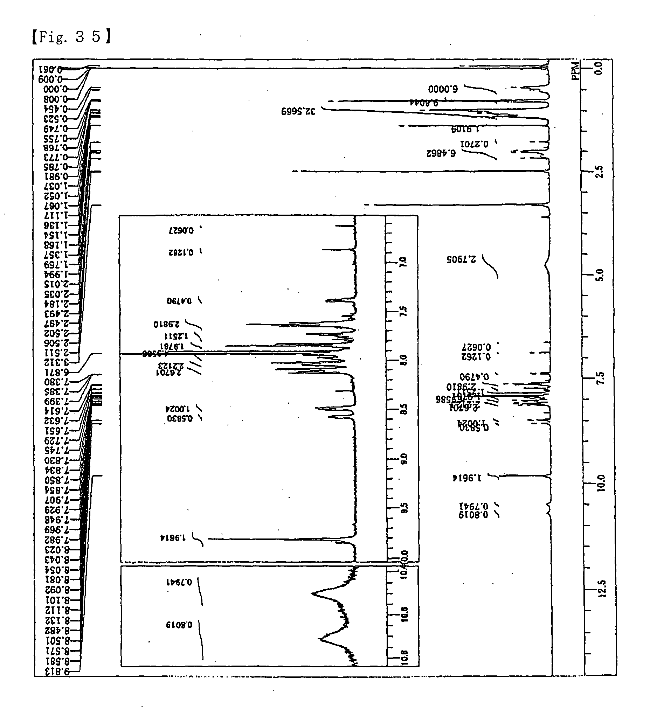

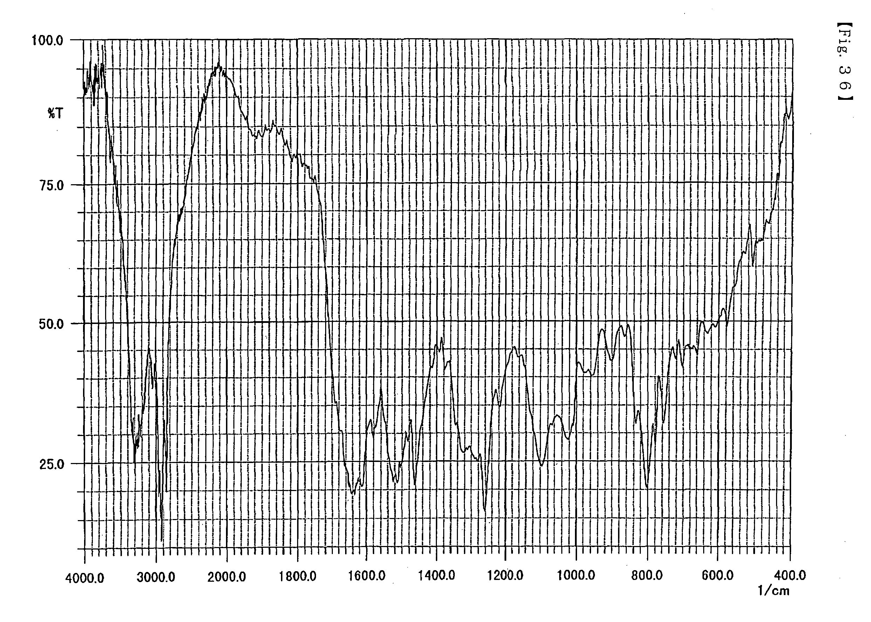

An NMR spectrum chart and an IR spectrum chart of the powder are respectively shown in Figure 35 and Figure 36. The powder was identified as a hydrazide having the structure represented by formula (22).

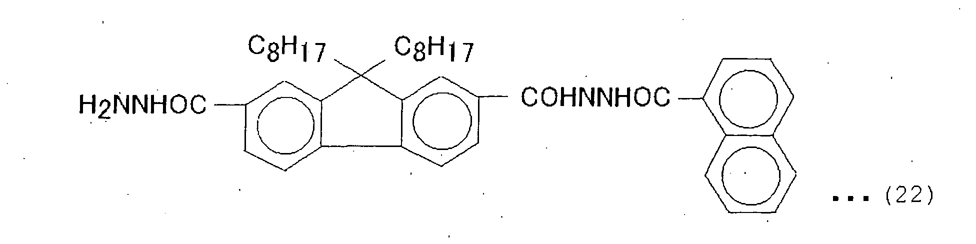

Next, in a 1L three-necked flask were placed 4.9 g of the hydrazide represented by formula (22) , 2.1 g of the compound represented by formula (8), which was also used in Example 1, 0.7 g of pyridine, and 290 ml of tetrahydrofuran. The solution in the three-necked flask was heated in a silicone oil bath to 110°C and allowed to react for 2 hours. After the completion of the reaction, the solution was introduced into ice water. The resultant was filtered with a suction funnel. The precipitated solids were collected, washed, and dried. 5 g of a solid matter was obtained.

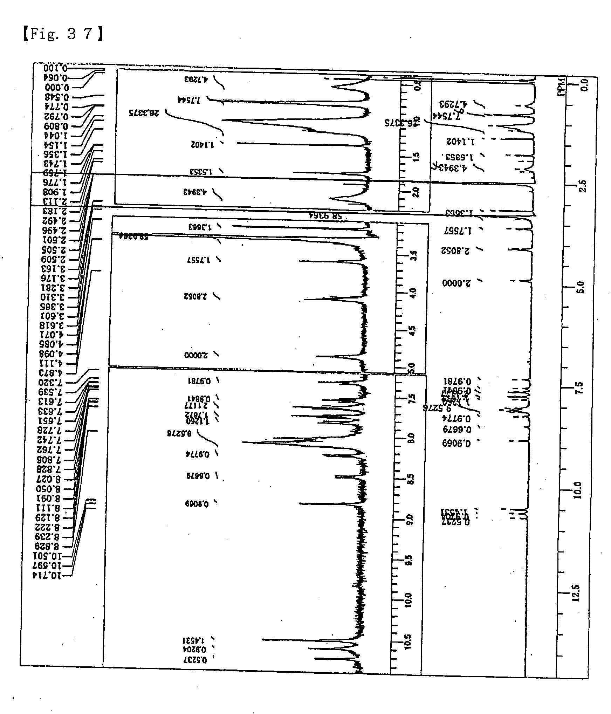

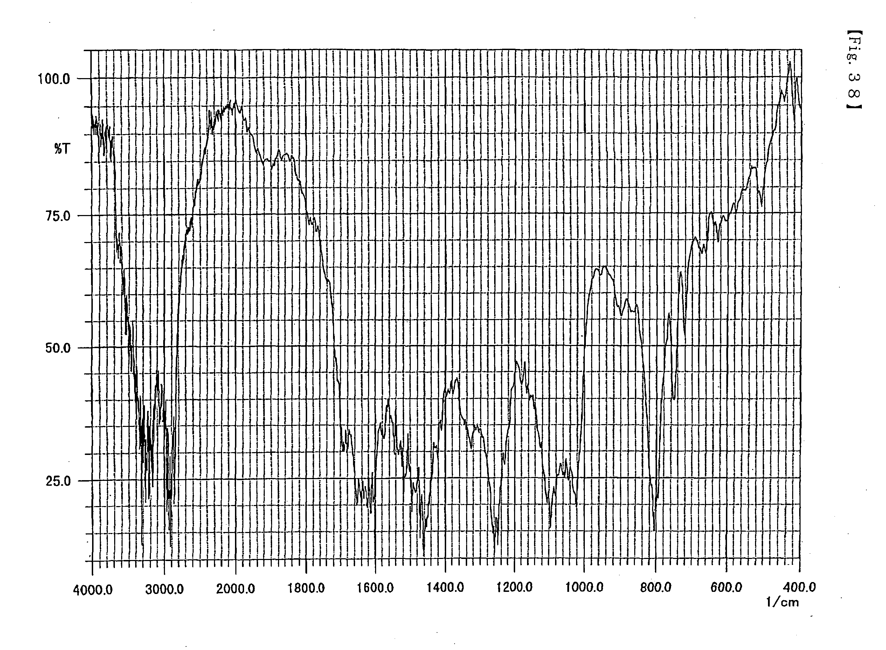

An NMR spectrum chart and an IR spectrum chart of the obtained solid matter are respectively shown in Figure 37 and Figure 38. The obtained was identified as a hydrazide having the structure represented by formula (23).

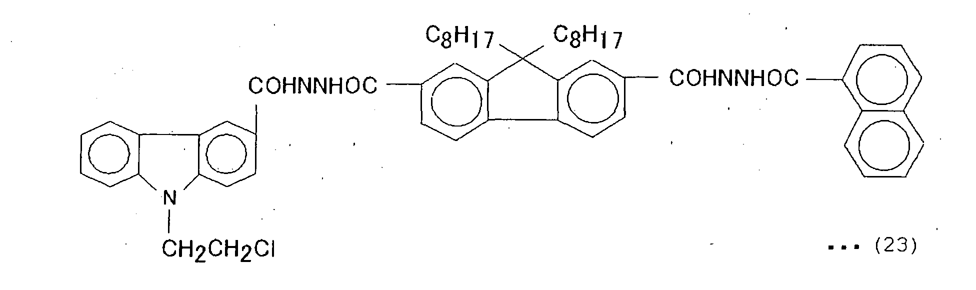

In a 500 ml pear-shaped flaskwere placed 5 g of the compound represented by formula (23), 200 ml of 1,4-dioxane, and 100 ml of phosphoryl chloride. The flask containing the solution was placed in a silicone oil bath and the solution was heated to 110°C. The reaction was allowed to continue for 14.5 hours. After the termination of the reaction, the contents in the flask were introduced into ice water. The resultant was filtered with a suction funnel. The solids obtained were collected, washed, and dried. 4.26 g of a solid matter was obtained.

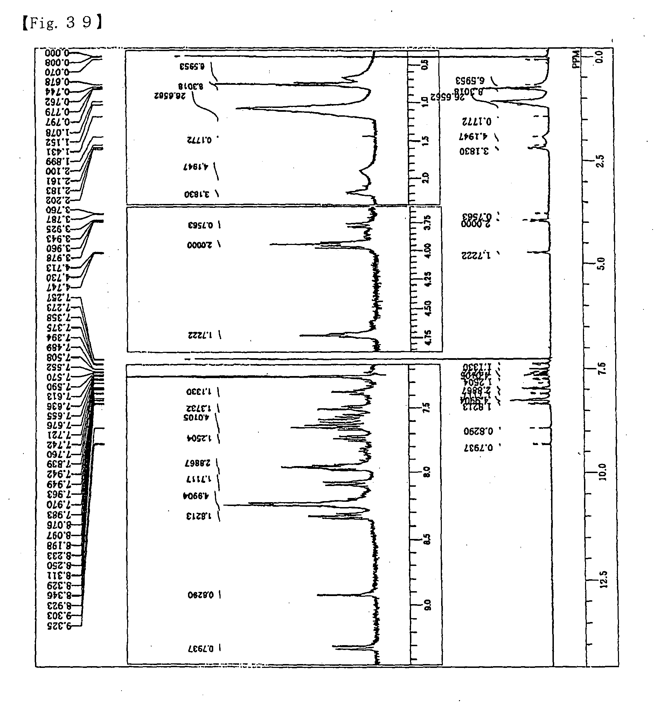

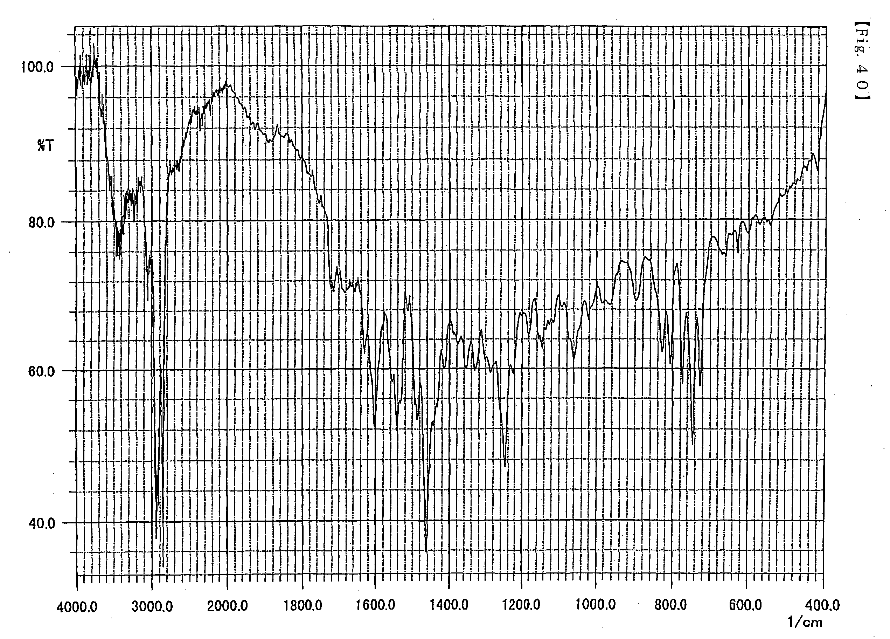

An NMR spectrum chart and an IR spectrum chart of the solid matter are respectively shown in Figure 39 and Figure 40. From these data, the solid matter was identified as a compound having the structure represented by formula (24).

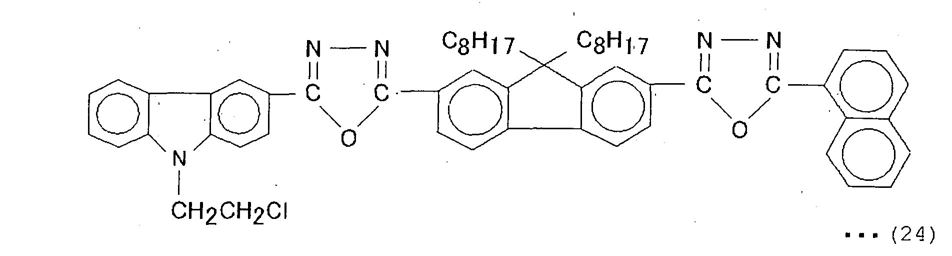

In a 500 ml pear-shaped flask were placed 0.34 g of the compound represented by formula (24), 0.7 g of potassium hydroxide, 50 ml of ethanol, and 100 ml of 1,4-dioxane. The flask containing the solution was placed in a silicone oil bath and the solution was heated to 100°C. The reaction was allowed to continue for 4 hours. After the termination of the reaction, the resultant solution was concentrated. The concentrate was cooled with ice and filtered with a suction funnel. Moisture in the filtrate obtained was removed with 50 g of anhydrous sodium sulfate, and the dehydrated filtrate was concentrated. Then, the concentrate was filtered. The solids obtained were washed with methanol, and dried to 0.23 g of white powder.

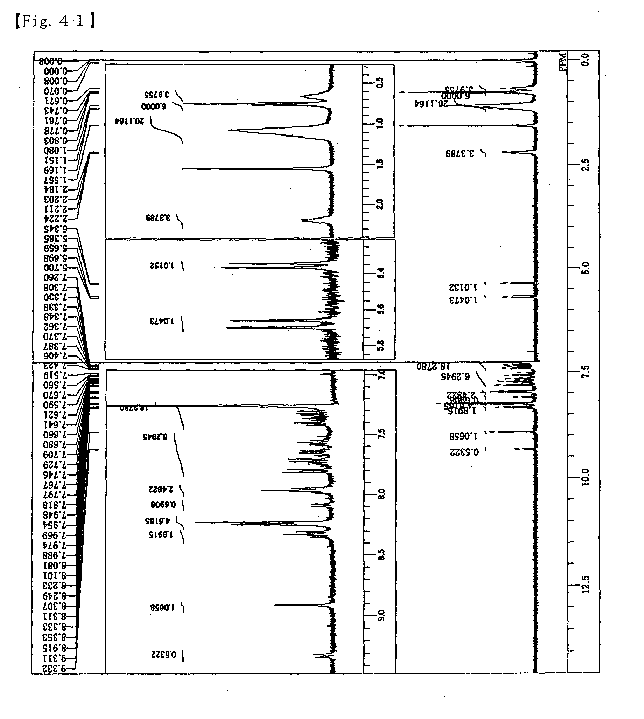

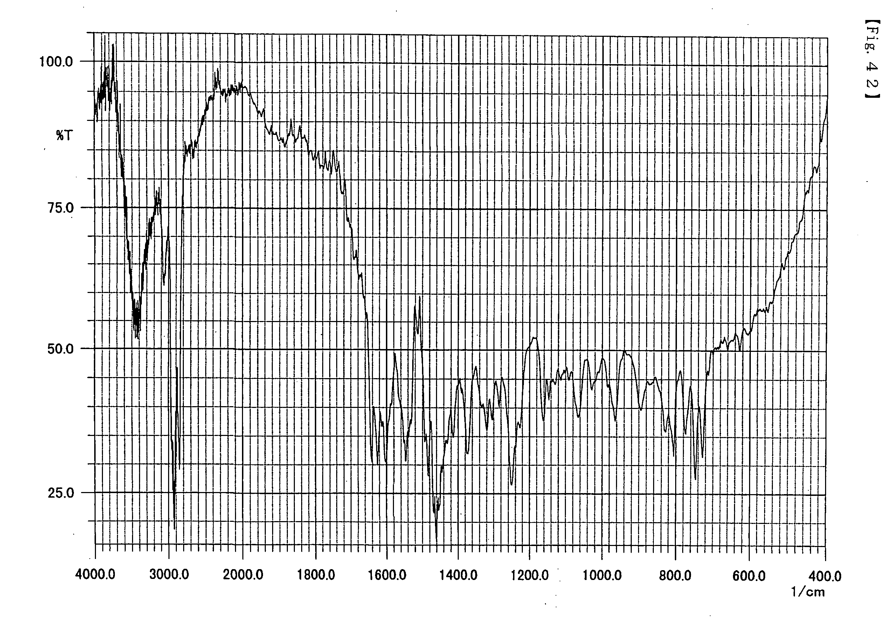

An NMR spectrum chart and an IR spectrum chart of the powder are respectively shown in Figure 41 and Figure 42. From these data, the powder was identified as a vinyl compound having the structure represented by formula (25).

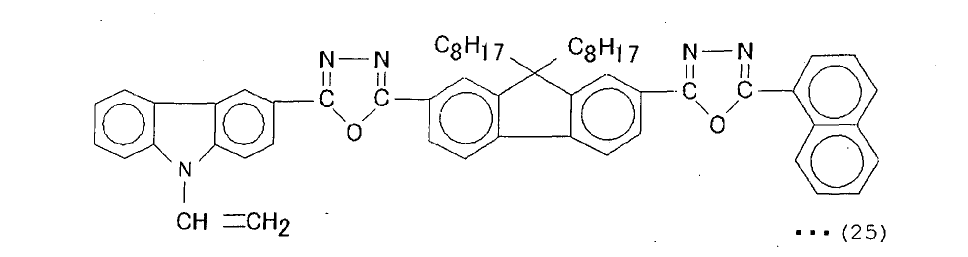

0.2 g of the vinyl compound represented by formula (25), 10 ml of dichlorobenzene, and 50 µl of a chlorobenzene solution including 2 wt% of tungsten hexachloride were placed in a 300 ml pear-shaped flask. The solution in the flask was shaken in a vibrating machine for 15 hours. Then, the contents in the flask were filtered. The solvent was distilled away from the filtrate, and the resultant was dried. 0.1 g of a light yellow solid matter was obtained.

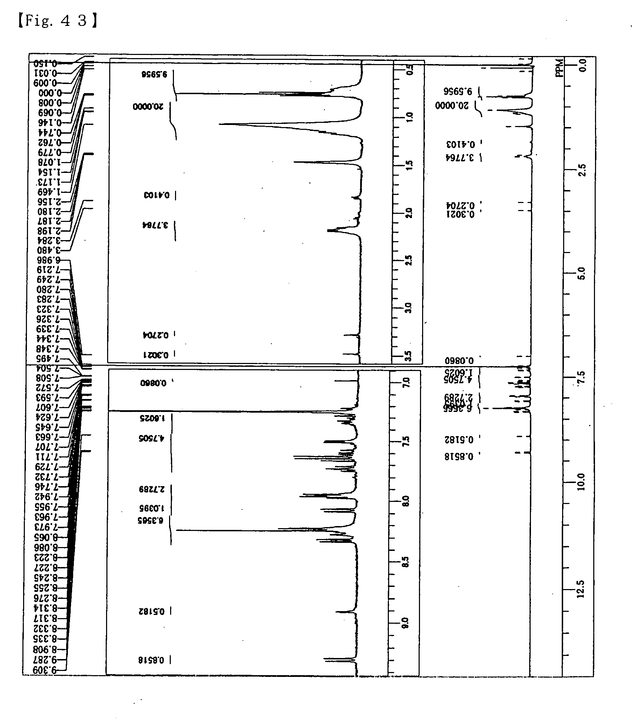

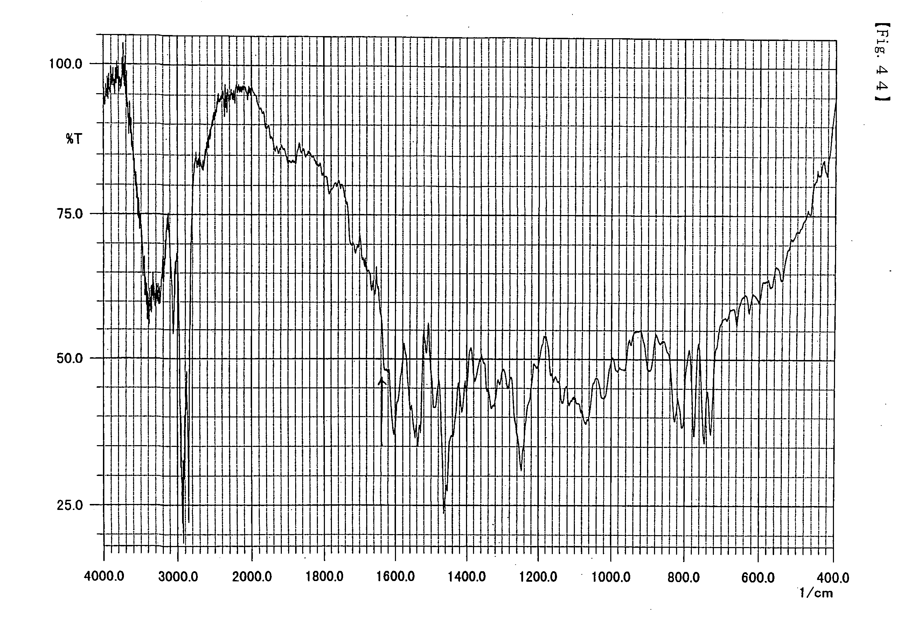

An NMR spectrum chart and an IR spectrum chart of the solid matter are respectively shown in Figure 43 and Figure 44. Fromthesedata, the solid matter was identified as a polymer having the repeating unit represented by formula (26).

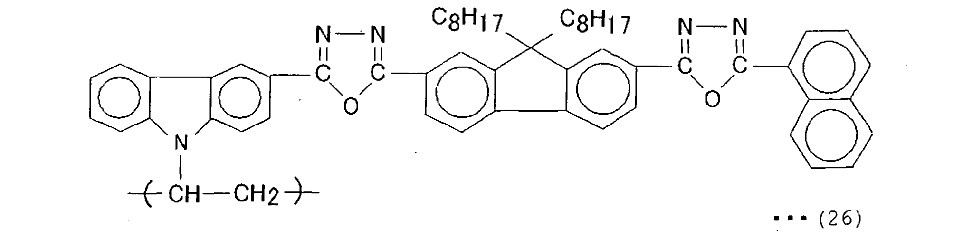

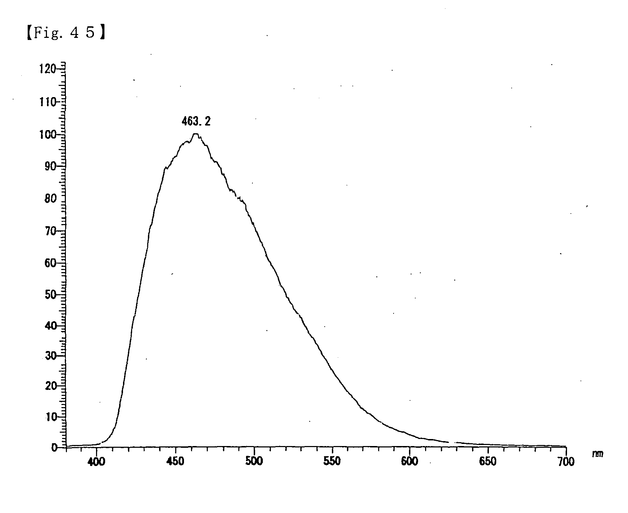

A fluorescence spectrum chart of this polymer is shown in Figure 45. This chart shows that the polymer obtained emits white light.

## Claims

1. A light-emitting compound having the structure represented by formula (1): 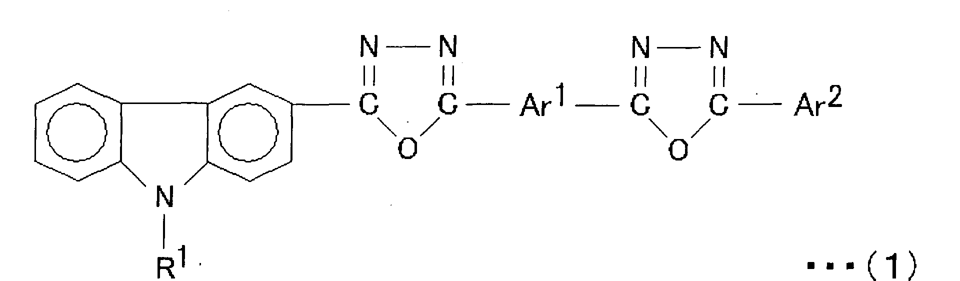 wherein R¹ is hydrogen atom, a vinyl group, an aryl group, or an alkyl group having 1 to 10 carbon atoms, hydrogen atoms of which may be replaced with halogen atoms; Ar¹ denotes a group made by eliminating two hydrogen atoms from an aromatic ring to which the hydrogen atoms are bonded; and Ar² denotes an aryl group.

2. The light-emitting compound as claimed in claim 1, wherein Ar¹ denotes a substituent shown by formula (2-1) or (2-2); and Ar² is an aryl group represented by formula (2-3) or (2-4): 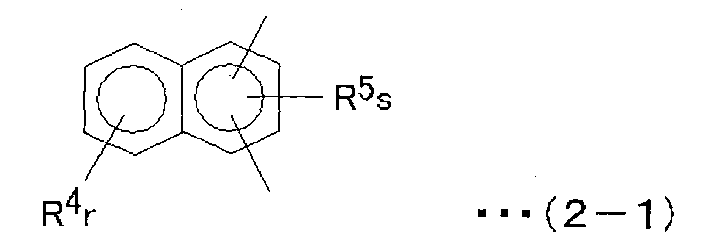 wherein each of R⁴ and R⁵ means hydrogen atom, or an alkyl group having 1 to 10 carbon atoms, hydrogen atoms of which may be replaced with halogen atoms; r denotes an integer of 1 to 4; and s denotes an integer of 1 or 2, wherein R⁴ and R⁵ may be the same or different from each other; R⁴s, the number of which number is r, may be the same or different from each other when r is not 1; and two R⁵s, when s is 2, may be the same or different from each other; 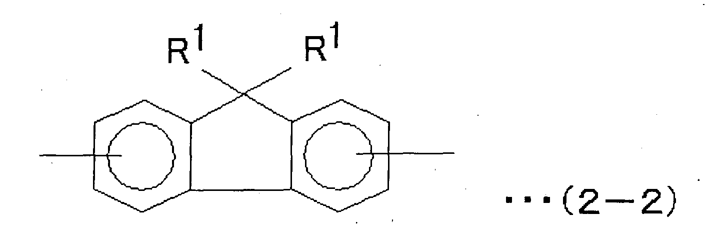 wherein R¹ means the same group as R¹ in formula (2); and two R¹s may be the same or different from each other; 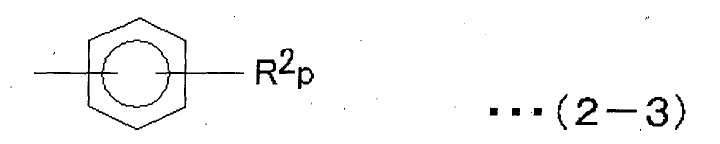 wherein R² means hydrogen atom, or an alkyl group having 1 to 10 carbon atoms, hydrogen atoms of which may be replaced with halogen atoms; p denotes an integer of 1 to 5; and R²s, the number of which is p, may be the same or different from each other when p is not 1; and 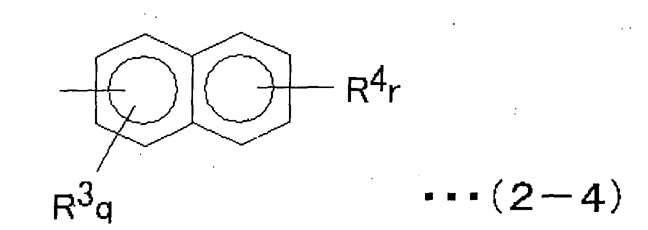 wherein R³ means hydrogen atom, or an alkyl group having 1 to 10 carbon atoms, hydrogen atoms of which may be replaced with halogen atoms; q denotes an integer of 1 to 3; R⁴ and r are the same as those defined in the explanation of formula (2-1) above; R³s, the number of which is q, may be the same or different from each other when q is not 1; and R⁴s, the number of which is r, may be the same or different from each other when r is not 1.

3. A light-emitting polymer having the repeating unit represented by formula (3): 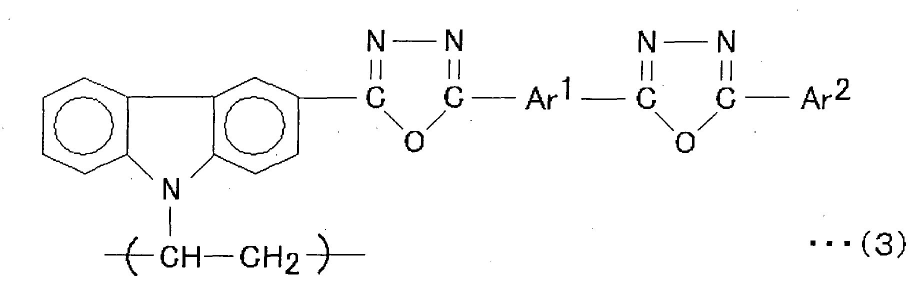 wherein Ar¹ denotes a group made by eliminating two hydrogen atoms from an aromatic ring to which the hydrogen atoms are bonded; and Ar² denotes an aryl group.

4. A layered article including the light-emitting compound of claim 1.

5. A layered article including the light-emitting compound of claim 2.

6. A layered article including the light-emitting polymer of claim 3.

7. A layered article according to claim 4 in a form of a luminescent' element comprising a pair of electrodes and a light-emitting layer sandwiched between the electrodes,
wherein the light-emitting compound is included in the light-emitting layer.

8. A layered article according to claim 5 in a form of a luminescent element comprising a pair of electrodes and a light-emitting layer sandwiched between the electrodes, wherein the light-emitting compound is included in the light-emitting layer.

9. A layered article according to claim 6 in a form of a luminescent element comprising a pair of electrodes and a light-emitting layer sandwiched between the electrodes,
wherein the light-emitting polymer is included in the light-emitting layer.
